# EUROPEAN PATENT SPECIFICATION

(11) **EP 4 539 783 B1**
(45) Date of publication and mention of the grant of the patent: **18.11.2026**
(21) Application number: 23741179.8
(22) Date of filing: 15.06.2023
(51) Int. Cl.: A61F 2/24, A61M 25/00

(54) **FLUID SEALING MECHANISM FOR A CATHETER**
FLÜSSIGKEITSABDICHTUNGSMECHANISMUS FÜR EINEN KATHETER
MÉCANISME D'ÉTANCHÉITÉ DE FLUIDE POUR UN CATHÉTER

(30) Priority: 16.06.2022 US 202263366517 P; 14.07.2022 US 202263368453 P; 15.08.2022 US 202263371463 P
(43) Date of publication of application: 23.04.2025
(73) Proprietor: Edwards Lifesciences Corporation, Irvine, CA 92614 (US)
(72) Inventor: REED, Kurt, Kelly, Irvine, CA 92614 (US); GANTZ, Kevin, Irvine, CA 92614 (US); PATEL, Darshin, S., Irvine, CA 92614 (US)
(74) Representative: Eisenführ Speiser
(86) International application number: PCT/US2023/025462
(87) International publication number: WO 2023/244751

(56) References cited:
- US-A1- 2006 030 923
- US-A1- 2014 277 403
- US-A1- 2015 164 669
- US-A1- 2017 065 368
- US-A1- 2021 138 210
- US-A1- 2021 393 405
- US-B2- 8 480 627

## Description

### CROSS-REFERENCE TO RELATED APPLICATIONS

This application claims the benefit of U.S. Provisional Application Nos. 63/366,517, filed June 16, 2022, 63/368,453, filed July 14, 2022, and 63/371,463, filed August 15, 2022.

### FIELD

The present disclosure relates to delivery apparatuses for docking devices configured to secure a prosthetic valve at a native heart valve.

### BACKGROUND

The human heart can suffer from various valvular diseases. These valvular diseases can result in significant malfunctioning of the heart and ultimately require repair of the native valve or replacement of the native valve with an artificial valve. There are a number of known repair devices (e.g., stents) and artificial valves, as well as a number of known methods of implanting these devices and valves in humans. Percutaneous and minimally-invasive surgical approaches are used in various procedures to deliver prosthetic medical devices to locations inside the body that are not readily accessible by surgery or where access without surgery is desirable. In one specific example, a prosthetic heart valve can be mounted in a crimped state on the distal end of a delivery apparatus and advanced through the patient's vasculature (e.g., through a femoral artery and the aorta) until the prosthetic valve reaches the implantation site in the heart. The prosthetic valve is then expanded to its functional size, for example, by inflating a balloon on which the prosthetic valve is mounted, actuating a mechanical actuator that applies an expansion force to the prosthetic valve, or by deploying the prosthetic valve from a sheath of the delivery apparatus so that the prosthetic valve can self-expand to its functional size.

Prosthetic heart valves may be appropriately sized to be placed inside many native aortic valves. However, native mitral and tricuspid valves can have a different geometry than typical aortic valves. Mitral and tricuspid valve anatomy can also vary significantly from person to person. Thus, it can be difficult to appropriately size and shape a prosthetic heart valve for use in a variety of patients. Further, when treating valve insufficiency, surrounding tissue at the target implantation site (e.g., native valve annulus) may not be strong enough to hold certain types of valves in position as desired.

In some examples, a docking device can be implanted first within the native valve and can be configured to receive a prosthetic heart valve and secure (e.g., anchor) the prosthetic heart valve in a desired position within the native valve. For example, the docking device can form a more circular and/or stable anchoring site at the native valve annulus in which a prosthetic heart valve can be expanded and implanted. A transcatheter delivery apparatus can be used to deliver the docking device to the implantation site. The docking device can be arranged within the delivery apparatus, coaxial with additional components of the delivery apparatus. Multiple lumens can be disposed between the coaxial components of the delivery apparatus, and a flush fluid may be provided to these lumens, before and during an implantation procedure, in order to flush and de-air the lumens. For example, the docking device can be covered by a sleeve shaft within an outer shaft of the delivery apparatus, and lumens can be formed between the outer shaft and sleeve shaft and between the sleeve shaft and docking device. In some instances, it may be desirable to de-air the sleeve shaft lumen in order to remove air around the docking device.

US 2014/277403 A1 relates to devices and methods for preparing a transcatheter heart valve system. The device includes a housing, sealing apparatuses and a port. The housing forms a chamber sized to receive a prosthetic valve and a portion of a delivery device. During use, the prosthesis is seated in the chamber, and the sealing apparatuses operated to seal the chamber about the delivery device. Air bubbles in a liquid delivered through the delivery device and to the chamber are removed from the system via the port, thereby flushing the system. Further, the device is manipulated to permit loading of the prosthesis into the delivery device in an air bubble-free environment.

US 2017/065368 A1 provides a cleaning device for a medical instrument including a flushing chamber, the flushing chamber having a lumen sized and configured to accept a distal end portion of the medical instrument during a cleaning process. The flushing chamber has an inlet port and an outlet port. Proximal and distal sealing members of the flushing chamber seal about the medical instrument when the instrument is inserted into the lumen of the flushing chamber.

A medical device which comprises a catheter having a catheter shaft, a sheath and a rolling membrane is known from US 2006/030923 A1. US 2015/164669 A1 provides a stent deployment system, handle, and method of loading of a medical device. US 2021/138210 A1 relates to a balloon catheter which allows for faster preparation and effective purging of air. US 8,480,627 B2 provides an introducer assembly with a handle assembly which enables automatic retraction and splitting of a sheath of the assembly. Methods, devices, and systems for performing endovascular repair of atrioventricular and other cardiac valves in the heart are known from US 2021/393405 A1.

### SUMMARY

The claimed invention is defined in independent claim 1 and relates to an assembly comprising, inter alia, a catheter and a sealing mechanism. The claimed invention also provides a method for flushing a catheter as defined in independent claim 12. Some preferred configurations of the claimed invention are defined in the dependent claims. Also described herein are related aspects, examples, embodiments and arrangements useful for understanding the claimed invention, and which do not necessarily constitute embodiments of the claimed invention. The subject-matter for which protection is sought is defined by the claims.

Described herein are docking devices, prosthetic heart valves, delivery apparatus, and methods for implanting docking devices and prosthetic heart valves within the docking devices. Also described herein are examples of flow mechanisms or assemblies that can be used to selectively direct fluid flow through lumens of a catheter in order to effectively flush and/or de-air specified lumens and/or components of the delivery apparatus. In some examples, the catheter is a portion of a delivery apparatus which comprises a docking device disposed within an outer shaft of the delivery apparatus and a sleeve shaft extending through the outer shaft and covering the docking device. The docking device can be configured to receive a prosthetic heart valve after being delivered at an implantation site using the delivery apparatus. The flow mechanisms or assemblies described herein can be coupled with a distal end portion of the delivery apparatus and configured to direct fluid flow through a lumen of the sleeve shaft, thereby de-airing the docking device prior to an implantation procedure.

A sealing mechanism can comprise a housing comprising a cavity and a step disposed within the cavity that decreases a diameter of the cavity from a larger diameter portion of the cavity to a smaller diameter portion of the cavity.

In some examples, the sealing mechanism can further comprise a first seal disposed within the housing adjacent and proximal to the larger diameter portion of the cavity, and a second seal disposed within the housing adjacent and distal to the smaller diameter portion of the cavity.

In some examples, the housing can comprise a first seal housing and a second seal housing, where the first seal is disposed in the first seal housing and the second seal is disposed in the second seal housing.

In some examples, the first seal is a compressible gasket, and the second seal is an O-ring.

In some examples, the first seal is an O-ring, and the second seal is an O-ring.

In some examples, the first seal and the second seal are annular, and an inner diameter of the first seal is larger than an inner diameter of the second seal.

In some examples, a sealing mechanism comprises a first seal housing with a first seal disposed within the first seal housing and a second seal housing with a second seal disposed within the second seal housing. A proximal portion of the second seal housing includes a step that transitions between a first diameter proximal to the step and a second diameter distal to the step, the second diameter smaller than the first diameter, and the step is disposed proximal to the second seal. The sealing mechanism further comprises a cavity defined within a distal portion of the first seal housing and the proximal portion of the second seal housing, between the first seal and the second seal.

In some examples, a sealing mechanism comprises a housing comprising a cavity and a step disposed within the cavity that decreases a diameter of the cavity from a larger diameter portion of the cavity to a smaller diameter portion of the cavity. The sealing mechanism further comprises a first seal disposed within the housing adjacent and proximal to the larger diameter portion of the cavity, and a second seal disposed within the housing adjacent and distal to the smaller diameter portion of the cavity.

In some examples, a sealing mechanism comprises a housing comprising a cavity and a step disposed within the cavity that decreases a diameter of the cavity from a larger diameter portion of the cavity to a smaller diameter portion of the cavity; a first seal disposed within the housing adjacent and proximal to the larger diameter portion of the cavity; and a second seal disposed within the housing adjacent and distal to the smaller diameter portion of the cavity.

In some examples, a sealing mechanism comprises a seal housing comprising a body portion, where an inner surface of the body portion defines a first cavity, and where the body portion comprises at least one curved slot that extends through the body portion, from an outer surface to the inner surface of the body portion. The seal housing further comprises a seal disposed within a portion of the first cavity of the seal housing, where the seal comprises a lumen configured for receiving a shaft assembly of a prosthetic implant delivery apparatus, a locking member comprising an outer wall and an inner wall with a second cavity defined therebetween, in a radial direction, where the body portion of the seal housing extends into and is rotatable within the second cavity of the locking member, and at least one pin coupled to the inner wall and configured to extend into and slide along the at least one curved slot. The seal housing and locking member are rotatable relative to one another between an unlocked configuration and a locked configuration. In the unlocked configuration the at least one pin is disposed at a first end of the at least one curved slot, and in the locked configuration the at least one pin is disposed at an opposing, second end of the at least one curved slot and the seal is compressed axially between the seal housing and the locking member such that a diameter of the lumen of the seal is decreased in the locked configuration relative to the unlocked configuration.

An assembly can comprise a catheter or a delivery apparatus, and a sealing mechanism.

In some examples, the catheter can comprise a first shaft and a second shaft extending through the first shaft.

In some examples, lumen is defined between an inner surface of the first shaft and an outer surface of the second shaft.

In some examples, the sealing mechanism can comprise a first seal disposed around a distal end portion of the first shaft, a second seal disposed around a portion of the second shaft that extends distal to the first shaft, and a cavity disposed within a housing of the sealing mechanism between the first seal and the second seal.

In some examples, a distal end of the first shaft is disposed within the cavity and the cavity is fluidly sealed by the first seal and the second seal.

In some examples, the assembly can further comprise an implantable medical device disposed within a distal end portion of the second shaft in a delivery configuration.

In some examples, the sealing mechanism can comprise first and second members that are pivotable relative to one another between an open and a closed configuration, where the first and second members are configured to receive the second shaft therebetween and seal around the second shaft when in the closed configuration.

In some examples, the sealing mechanism can comprise a first member and a second member that are pivotable relative to one another between an open and a closed configuration, where the first and second members are configured to receive the second shaft therebetween and seal around the second shaft when in the closed configuration.

According to the invention as defined in independent claim 1, an assembly comprises a catheter, the catheter comprising a first shaft and a second shaft extending through the first shaft. A first lumen is defined between an inner surface of the first shaft and an outer surface of the second shaft. The assembly further comprises a sealing mechanism comprising a first seal disposed around a distal end portion of the first shaft, a second seal disposed around a portion of the second shaft that extends distal to the first shaft, and a cavity disposed within a housing of the sealing mechanism between the first seal and the second seal. A distal end of the first shaft is disposed within the cavity, and the cavity is fluidly sealed by the first seal and the second seal such that fluid from the first lumen cannot exit the cavity.

In some examples, an assembly comprises a delivery apparatus. The delivery apparatus comprises a first shaft, second shaft extending through the first shaft, where a first lumen is defined between an inner surface of the first shaft and an outer surface of the second shaft and a second lumen is defined by the second shaft, where the first and second lumens are fluidly coupled to one another. The assembly further comprises an implantable medical device disposed within a distal end portion of the second shaft in a delivery configuration, and a sealing mechanism. The sealing mechanism comprises a housing, a first seal disposed within the housing and around a distal end portion of the first shaft, a second seal disposed within the housing and around the distal end portion of the second shaft, and a cavity disposed within the housing and defined between the first seal and the second seal. A distal end of the first shaft is disposed within the cavity, a distal end of the second shaft extends distal to the distal end of the first shaft and the second seal, and the cavity is fluidly sealed by the first seal and the second seal.

In some examples, an assembly comprises a catheter, the catheter comprising a first shaft and a second shaft extending through the first shaft, where a distal end portion of the second shaft is extendable distal to a distal end of the first shaft. The assembly further comprises a sealing mechanism, the sealing mechanism comprising first and second members that are pivotable relative to one another between an open and a closed configuration, where the first and second members are configured to receive the second shaft therebetween and seal around the second shaft when in the closed configuration. The sealing mechanism further comprises a tube fluidly connected to a lumen defined by the first and second members. An end of the tube comprises an attachment configured to receive an aspiration tool for aspirating fluid through the second shaft.

In some examples, an assembly comprising: a catheter comprising: a first shaft; and a second shaft extending through the first shaft, wherein a distal end portion of the second shaft is extendable distal to a distal end of the first shaft; and a sealing mechanism comprising: a first member and a second member that are pivotable relative to one another between an open and a closed configuration, wherein the first and second members are configured to receive the second shaft therebetween and seal around the second shaft when in the closed configuration; and a tube fluidly connected to a lumen defined by the first and second members, and wherein an end of the tube comprises an attachment configured to receive an aspiration tool for aspirating fluid through the second shaft.

In some examples, an assembly comprises a catheter comprising a first shaft and a second shaft extending through the first shaft, where a distal end portion of the second shaft is extendable distal to a distal end of the first shaft. The assembly further comprises a sealing mechanism comprising a seal disposed around a distal end portion of the second shaft, and a seal housing comprising a cylindrical body portion, where an inner surface of the cylindrical body portion defines a first cavity, and where the seal is disposed within the first cavity. The sealing mechanism further comprises a locking member comprising an annular outer wall and an annular inner wall with a second cavity defined therebetween, in a radial direction, where the cylindrical body portion extends into and is rotatable within the second cavity, and where the seal housing and the locking member are configured to receive the second shaft therethrough. The seal housing and locking member are rotatable relative to one another between an unlocked configuration and a locked configuration. In the locked configuration the seal is compressed axially between the seal housing and the locking member and compressed radially around the second shaft.

In some examples, an assembly comprises one or more of the components recited in Examples 1-20, 54-69, and 80-97 below.

A method for flushing a catheter can comprise positioning a first seal of a sealing mechanism around a distal end portion of a first shaft of a catheter, positioning a second seal of a sealing mechanism around a distal end portion of a second shaft of the catheter that extends through the first shaft, and flowing fluid through the catheter such that the fluid flows out of only a second lumen defined by the second shaft.

In some examples, the distal end portion of the second shaft extends distal to a distal end of the first shaft.

In some examples, the method includes tightening the first seal around the distal end portion of the first shaft and the second seal around the distal end portion of the second shaft.

In some examples, the flowing fluid through the catheter can further comprise blocking fluid from flowing out of a second lumen defined between an outer surface of the second shaft and an inner surface of the first shaft.

According to the invention as defined in independent claim 12, method for flushing a catheter comprises attaching a first seal of a sealing mechanism to a distal end portion of a first shaft of a catheter, attaching a second seal of a sealing mechanism to a distal end portion of a second shaft of the catheter that extends through the first shaft, where the distal end portion of the second shaft extends distal to a distal end of the first shaft, and flowing fluid through the catheter such that the fluid flows out of only a second lumen defined by the second shaft and is blocked from flowing out of a first lumen defined between an outer surface of the second shaft and an inner surface of the first shaft.

In some examples, a method for flushing a catheter comprises extending a distal end portion of a first shaft of a catheter through a first seal disposed in a first seal housing of a sealing mechanism and into a cavity disposed within the first seal housing and a second seal housing of the sealing mechanism, the cavity defined between the first seal and a second seal of the second seal housing. The method further comprises extending a distal end portion of a second shaft of the catheter through and distal to a distal end of the first shaft and through the second seal disposed within the second seal housing, tightening the first seal around the distal end portion of the first shaft and the second seal around the distal end portion of the second shaft, and flowing fluid through the catheter such that the fluid flows out of only a first lumen defined by the second shaft and is blocked from flowing out of a second lumen defined between an outer surface of the second shaft and an inner surface of the first shaft.

The various innovations of this disclosure can be used in combination or separately. This summary is provided to introduce a selection of concepts in a simplified form that are further described below in the detailed description. The foregoing and other objects, features, and advantages of the disclosure will become more apparent from the following detailed description, claims, and accompanying figures.

### BRIEF DESCRIPTION OF THE DRAWINGS

FIG. 1 schematically illustrates a first stage in an exemplary mitral valve replacement procedure where a guide catheter and a guidewire are inserted into a blood vessel of a patient and navigated through the blood vessel and into a heart of the patient, towards a native mitral valve of the heart.
FIG. 2A schematically illustrates a second stage in the exemplary mitral valve replacement procedure where a docking device delivery apparatus extending through the guide catheter is implanting a docking device for a prosthetic heart valve at the native mitral valve.
FIG. 2B schematically illustrates a third stage in the exemplary mitral valve replacement procedure where the docking device of FIG. 2A is fully implanted at the native mitral valve of the patient and the docking device delivery apparatus has been removed from the patient.
FIG. 3A schematically illustrates a fourth stage in the exemplary mitral valve replacement procedure where a prosthetic heart valve delivery apparatus extending through the guide catheter is implanting a prosthetic heart valve in the implanted docking device at the native mitral valve.
FIG. 3B schematically illustrates a fifth stage in the exemplary mitral valve replacement procedure where the prosthetic heart valve is fully implanted within the docking device at the native mitral valve and the prosthetic heart valve delivery apparatus has been removed from the patient.
FIG. 4 schematically illustrates a sixth stage in the exemplary mitral valve replacement procedure where the guide catheter and the guidewire have been removed from the patient.
FIG. 5 is a side perspective view of a docking device in a helical configuration, according to one example.
FIG. 6 is a side view of an exemplary delivery apparatus for a docking device, the delivery apparatus including a handle assembly and an outer shaft extending distally from the handle assembly, the outer shaft configured to contain a docking device therein in a delivery configuration.
FIG. 7 is a perspective view of a distal end portion of the delivery apparatus of FIG. 6, which illustrates an exemplary docking device deployed from an outer shaft of the delivery apparatus and covered by a sleeve shaft of the delivery apparatus.
FIG. 8 is a perspective view of a distal end portion of the delivery apparatus of FIG. 6, which illustrates the exemplary docking device of FIG. 7 deployed from the outer shaft of the delivery apparatus with the sleeve shaft removed from the docking device.
FIG. 9 is a schematic cross-sectional view of the delivery apparatus of FIG. 6, which illustrates fluid flow from a first flushing port through multiple fluidly connected lumens of the delivery apparatus.
FIG. 10 is another schematic cross-sectional view of the delivery apparatus of FIG. 6, which illustrates fluid flow from a second flushing port through multiple fluidly connected lumens of the delivery apparatus.
FIG. 11 is a side view of an exemplary sealing mechanism for a catheter that is configured to regulate fluid flow through two shafts of the catheter, the sealing mechanism shown coupled to an outer shaft and sleeve shaft of the delivery apparatus of FIG. 6.
FIG. 12 is a first end view of the sealing mechanism of FIG. 11.
FIG. 13 is an opposite, second end view of the sealing mechanism of FIG. 11.
FIG. 14 is a cross-sectional side view of the sealing mechanism of FIG. 11, showing the sealing mechanism coupled to the outer shaft and the sleeve shaft of the delivery apparatus of FIG. 6.
FIG. 15 shows a cross-sectional perspective view of the sealing mechanism of FIG. 11.
FIG. 16 shows an exploded view of the sealing mechanism of FIG. 11.
FIG. 17 is a flow chart of a method for selectively directing fluid flow through a catheter comprising multiple shafts that are at least partially concentric with one another using a sealing mechanism.
FIG. 18 is a perspective view of an exemplary sealing mechanism for a catheter that is configured to regulate fluid flow through two shafts of the catheter, the sealing mechanism comprising one compressible seal and one O-ring seal.
FIG. 19 is an exploded view of the sealing mechanism of FIG. 18.
FIG. 20 is a cross-sectional side view of the sealing mechanism of FIG. 18.
FIG. 21 is another cross-sectional side view of the sealing mechanism of FIG. 18, showing the sealing mechanism coupled to the outer shaft and the sleeve shaft of the delivery apparatus of FIG. 6.
FIG. 22 is a perspective view of an exemplary sealing mechanism for a catheter that is configured to regulate fluid flow through two shafts of the catheter, the sealing mechanism comprising two O-ring seals.
FIG. 23 is a cross-sectional side view of the sealing mechanism of FIG. 22.
FIG. 24 is a cross-sectional side view of the sealing mechanism of FIG. 18 which further includes an additional cavity and attachment for aspirating a shaft of a catheter.
FIG. 25 is a perspective view of an exemplary sealing mechanism for sealing to a shaft of a catheter and aspirating fluid out of the shaft.
FIG. 26 is a perspective view of the sealing mechanism of FIG. 25 in a closed configuration.
FIG. 27 is a perspective view of a sealing mechanism for sealing to a shaft of a catheter and aspirating fluid out of or flushing fluid through the shaft.
FIG. 28 is an exploded view of the sealing mechanism of FIG. 27.
FIG. 29A is a first perspective view of a locking cap of the sealing mechanism of FIG. 27.
FIG. 29B is a second perspective view of the locking cap of FIG. 29A.
FIG. 29C is a side view of the locking cap of FIG. 29A.
FIG. 30A is side perspective view of a seal housing of the sealing mechanism of FIG. 27.
FIG. 30B is an end perspective view of the seal housing of FIG. 27.
FIG. 31 is a side view of a seal of the sealing mechanism of FIG. 27.
FIG. 32A is a side view of the sealing mechanism of FIG. 27 in an unlocked configuration.
FIG. 32B is a side view of the sealing mechanism of FIG. 27 in a locked configuration.
FIG. 33A is a cross-sectional side view of the sealing mechanism of FIG. 32A in the unlocked configuration.
FIG. 33B is a cross-sectional side view of the sealing mechanism of FIG. 32B in the locked configuration.
FIG. 34 is a perspective view of the sealing mechanism coupled to an aspiration tool and a shaft of a catheter to be aspirated.

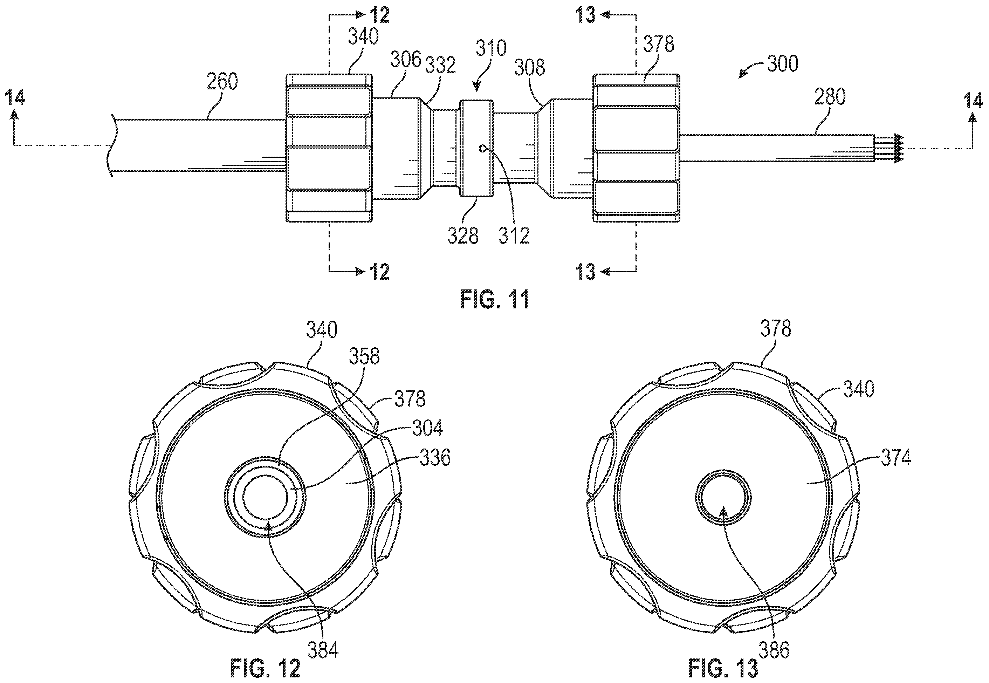

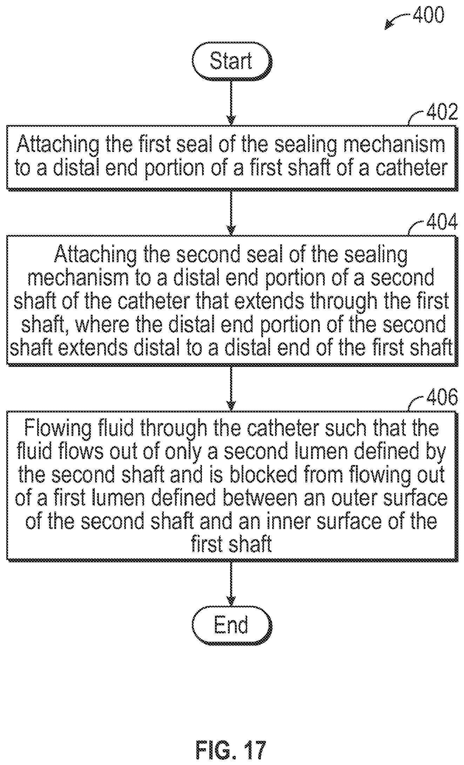

Specific embodiments of the invention defined in independent claim 1 are illustrated in Figs. 11-16 and 18-24. An embodiment of the invention defined in independent claim 12 is illustrated in Fig. 17.

### DETAILED DESCRIPTION

### General Considerations

For purposes of this description, certain aspects, advantages, and novel features of examples of this disclosure are described herein. The disclosed methods, apparatus, and systems should not be construed as being limiting in any way. Instead, the present disclosure is directed toward all novel and nonobvious features and aspects of the various disclosed examples, alone and in various combinations and sub-combinations with one another. The methods, apparatus, and systems are not limited to any specific aspect or feature or combination thereof, nor do the disclosed examples require that any one or more specific advantages be present or problems be solved.

Although the operations of some of the disclosed examples are described in a particular, sequential order for convenient presentation, it should be understood that this manner of description encompasses rearrangement, unless a particular ordering is required by specific language set forth below. For example, operations described sequentially may in some cases be rearranged or performed concurrently. Moreover, for the sake of simplicity, the attached figures may not show the various ways in which the disclosed methods can be used in conjunction with other methods. Additionally, the description sometimes uses terms like "provide" or "achieve" to describe the disclosed methods. These terms are high-level abstractions of the actual operations that are performed. The actual operations that correspond to these terms may vary depending on the particular implementation and are readily discernible by one of ordinary skill in the art.

As used in this application and in the claims, the singular forms "a," "an," and "the" include the plural forms unless the context clearly dictates otherwise. Additionally, the term "includes" means "comprises." Further, the term "coupled" generally means physically, mechanically, chemically, magnetically, and/or electrically coupled or linked and does not exclude the presence of intermediate elements between the coupled or associated items absent specific contrary language.

As used herein, the term "proximal" refers to a position, direction, or portion of a device that is closer to the user and further away from the implantation site. As used herein, the term "distal" refers to a position, direction, or portion of a device that is further away from the user and closer to the implantation site. Thus, for example, proximal motion of a device is motion of the device away from the implantation site and toward the user (e.g., out of the patient's body), while distal motion of the device is motion of the device away from the user and toward the implantation site (e.g., into the patient's body). The terms "longitudinal" and "axial" refer to an axis extending in the proximal and distal directions, unless otherwise expressly defined.

As used herein, "e.g." means "for example," and "i.e." means "that is."

### Introduction to the Disclosed Technology

As introduced above, a delivery apparatus can be used to deliver a docking device for a prosthetic heart valve to a target implantation site (e.g., a native valve annulus). The docking device can be arranged within a distal end portion of an outer shaft of the delivery apparatus in a relatively straight (e.g., uncoiled) delivery configuration. In some instances, a portion of the docking device can include an outer guard member that is collapsible and expandable. Additionally, a sleeve shaft of the delivery apparatus can extend through the outer shaft and be disposed around (and cover) the docking device. Multiple lumens are formed within the delivery apparatus, including a first lumen between the outer shaft and sleeve shaft and a second lumen within the sleeve shaft (e.g., between the sleeve shaft and docking device). These lumens can be flushed and de-aired prior to introduction of the delivery apparatus into a patient. However, since the first and second lumens are fluidly coupled with one another, flushing fluid applied to one or more of these lumens may not result in enough flushing pressure to be applied to the second lumen such that the guard member of the docking device is sufficiently de-aired. Accordingly, improvements to flushing and de-airing procedures for catheters and delivery apparatus having multiple fluidly connected lumens are desirable. Such improvements can, for example, enable the sleeve shaft lumen and the docking device to be effectively and fully de-aired prior to an implantation procedure.

Described herein are various systems, apparatuses, methods, or the like, that, in some examples, can be used in or with delivery apparatuses for prosthetic medical devices (such as docking devices for prosthetic heart valves). In some examples, such systems, apparatuses, and/or methods can provide a system and/or method for selectively directing fluid flow through a catheter (e.g., a delivery apparatus) comprising multiple shafts that are at least partially concentric with one another (or one shaft arranged at least partially within another shaft) in order to flush and de-air specified lumens of the catheter.

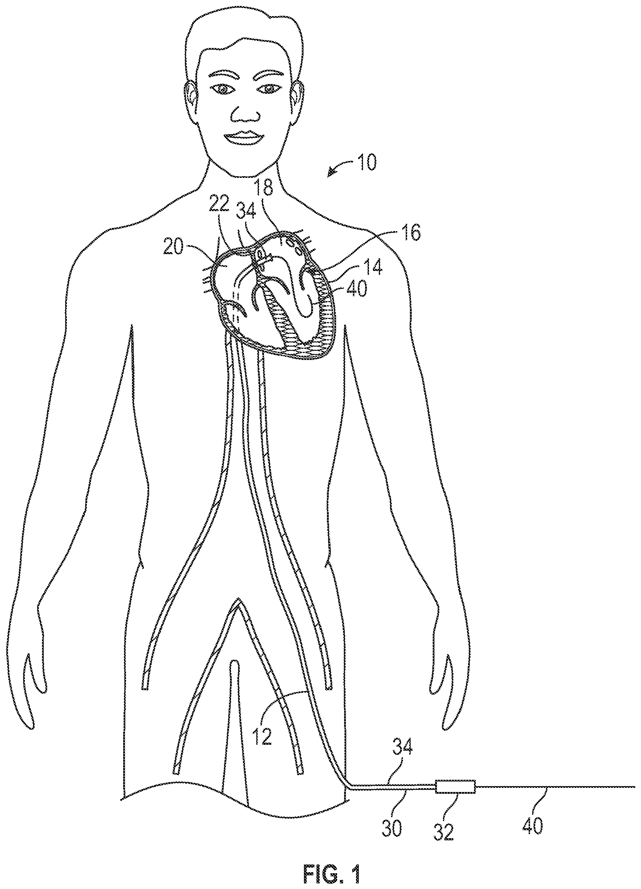

In some examples, the docking device delivery apparatuses disclosed herein can be used to deliver a docking device to a target implantation site in a patient. For example, FIGS. 1-4 schematically illustrate an exemplary transcatheter heart valve replacement procedure which utilizes a guide catheter to guide a docking device delivery apparatus toward a native valve annulus and then a prosthetic heart valve delivery apparatus toward the native valve annulus. The docking device delivery apparatus is used to deliver a docking device to the native valve annulus and then the prosthetic heart valve delivery apparatus is used to deliver a transcatheter prosthetic heart valve inside the docking device.

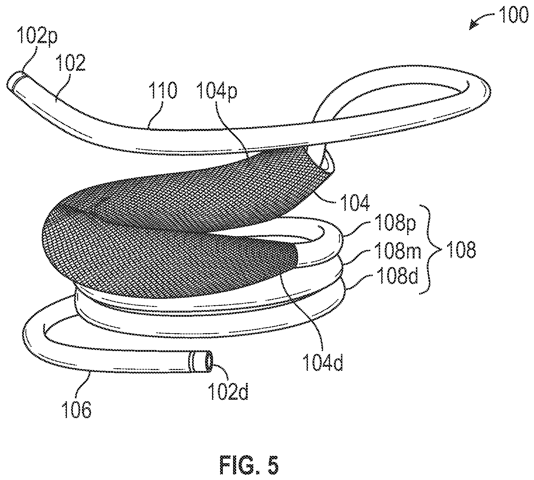

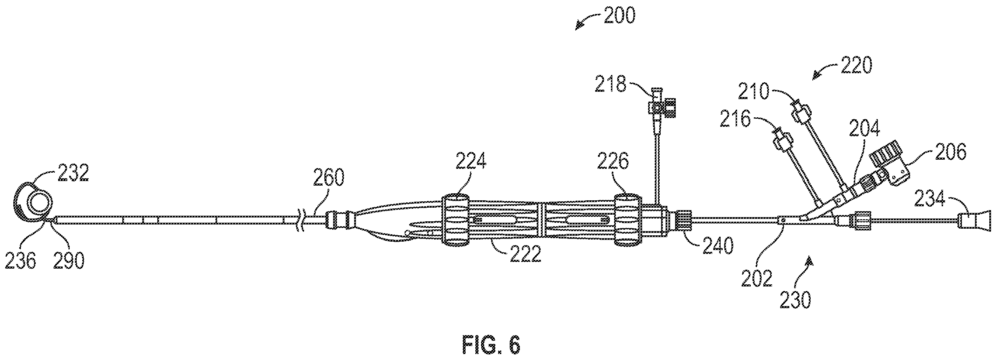

As introduced above, defective native heart valves may be replaced with transcatheter prosthetic heart valves. However, such prosthetic heart valves may not be able to sufficiently conform to the geometry of the native tissue (e.g., to the leaflets and/or annulus of the native heart valve) and may undesirably shift around relative to the native tissue, which can lead to paravalvular leakage. Thus, a docking device may be implanted first at the native valve annulus and then the prosthetic heart valve can be implanted within the docking device to help anchor the prosthetic heart valve to the native tissue and provide a seal between the native tissue and the prosthetic heart valve. An exemplary docking device is shown in FIG. 5 and an exemplary delivery apparatus for deploying the docking device at a native heart valve is shown in FIG. 6.

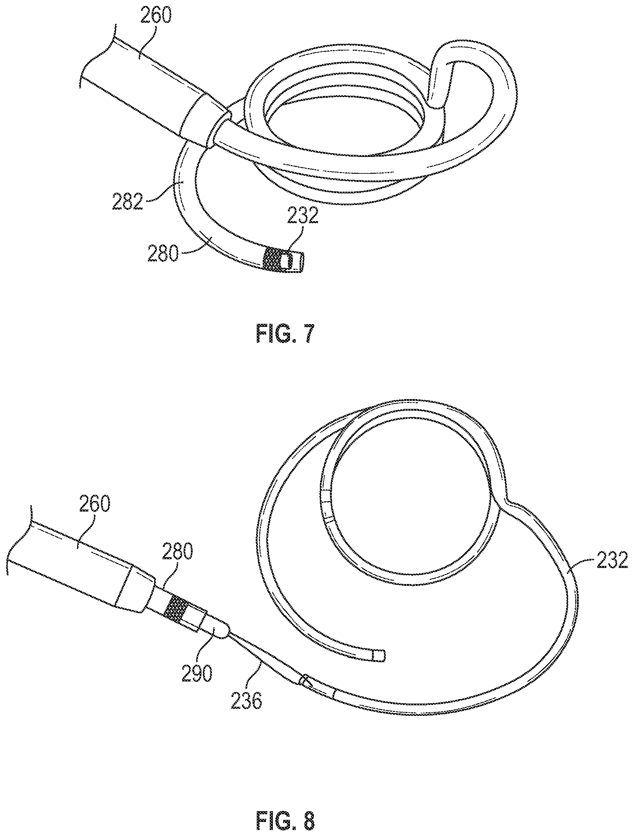

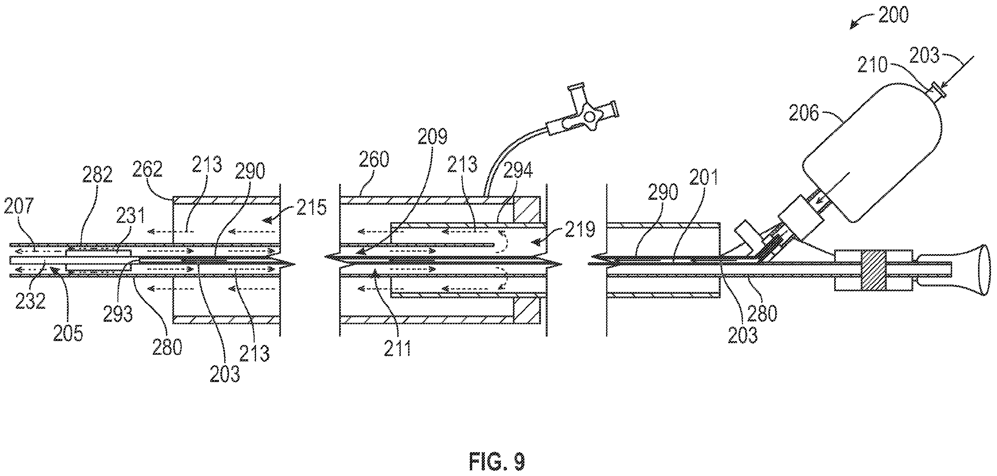

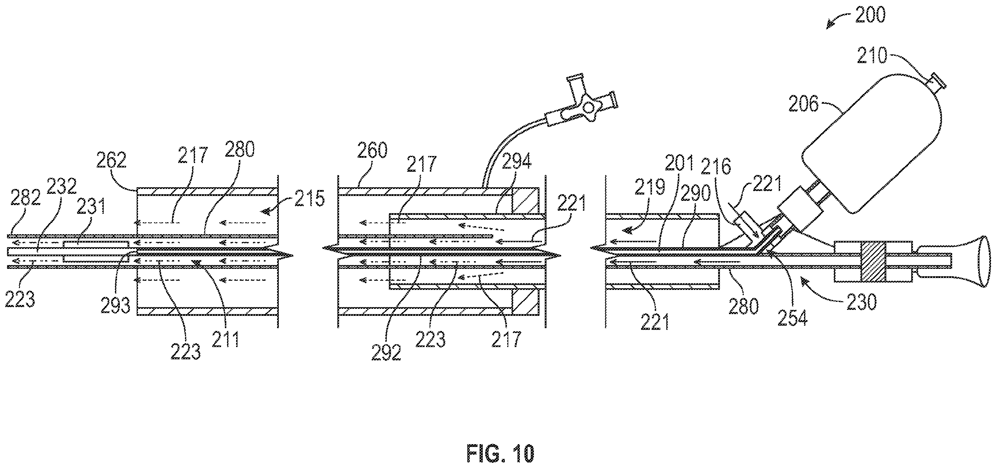

As shown in FIGS. 7-10, the docking device delivery apparatus can comprise an outer shaft, a sleeve shaft extending through the outer shaft and containing a docking device therein in a relatively straight delivery configuration, and a pusher shaft extending through the outer shaft and disposed adjacent to a proximal end of the docking device. Multiple lumens are formed in the delivery apparatus, including a sleeve shaft lumen through the sleeve shaft and an outer shaft lumen formed between the outer shaft and the sleeve shaft. These lumens can be fluidly coupled to one another, and thus, fluid flow through one of the lumens can also enter the other one of the lumens during a flushing process, as illustrated schematically in FIGS. 9 and 10.

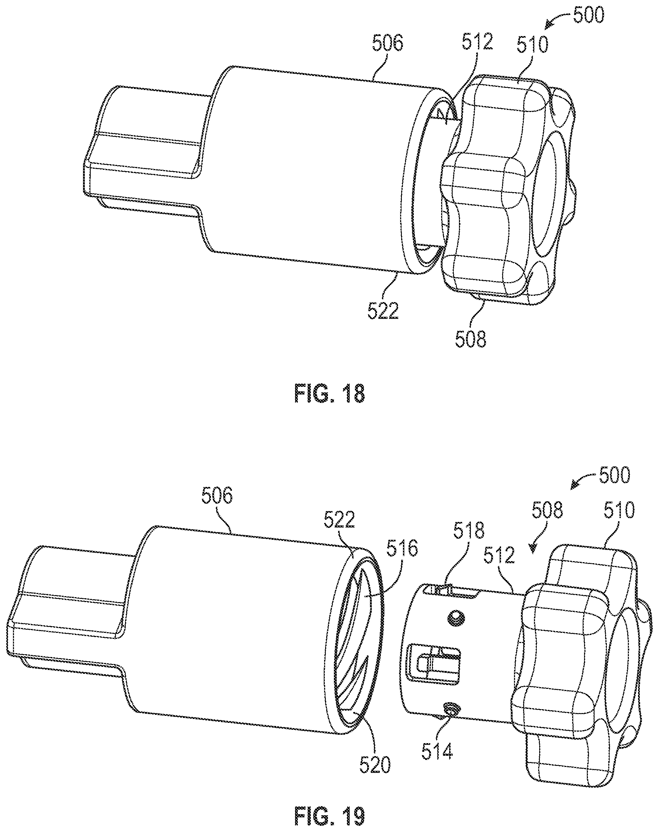

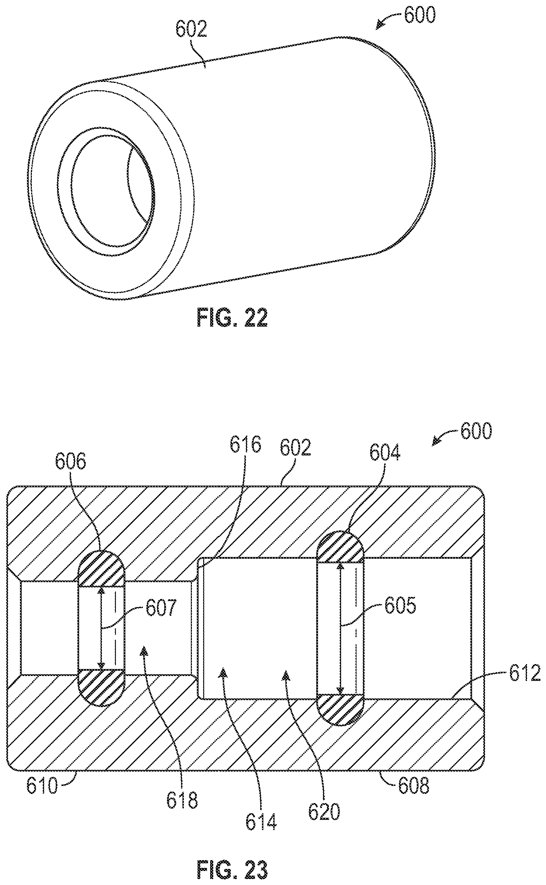

In some examples, as shown in FIGS. 11-16, a sealing mechanism (or assembly) comprising two seals can be configured to receive a distal end portion of the outer shaft and the sleeve shaft therethrough. The sealing mechanism can be configured to seal (e.g., with a first seal) around an outer surface of the outer shaft and seal (e.g., with a second seal disposed distal to the first seal) around an outer surface of the sleeve shaft which extends distal to a distal end of the outer shaft. As a result, flushing fluid entering the lumens of the delivery apparatus can be blocked from exiting the outer shaft lumen, which thereby forces all or a majority of the flushing fluid to flow through the sleeve shaft lumen. As a result, the sleeve shaft lumen and docking device disposed therein can be effectively and fully flushed and de-aired. In some instances, the seals can both be compressible seals or gaskets (FIGS. 11-16). In some instances, the seal around the outer shaft can be a compressible seal or gasket and the seal around the sleeve shaft can be an O-ring (FIGS. 18-21). In some instances, both the seals can be O-rings of different sizes (FIGS. 22-23).

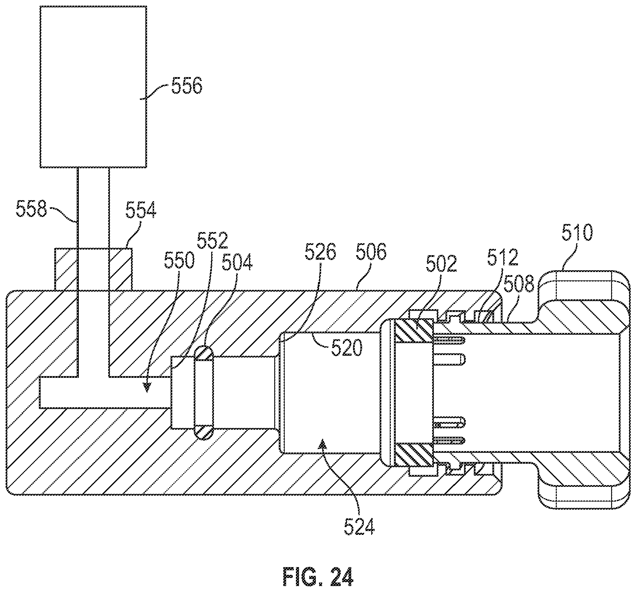

In some examples, instead of flushing the sleeve shaft (or an alternate shaft of a catheter) using the sealing mechanism, any of the sealing mechanisms described above can be used to seal around the outer shaft and sleeve shaft (or an inner and outer shaft of an alternate catheter) and aspirate the sleeve shaft using an aspiration tool (such as a syringe) (FIG. 24).

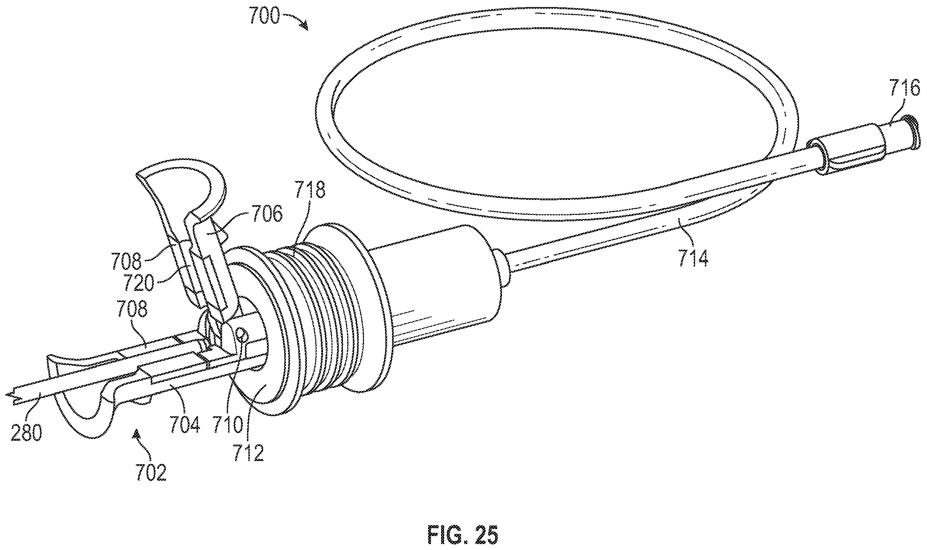

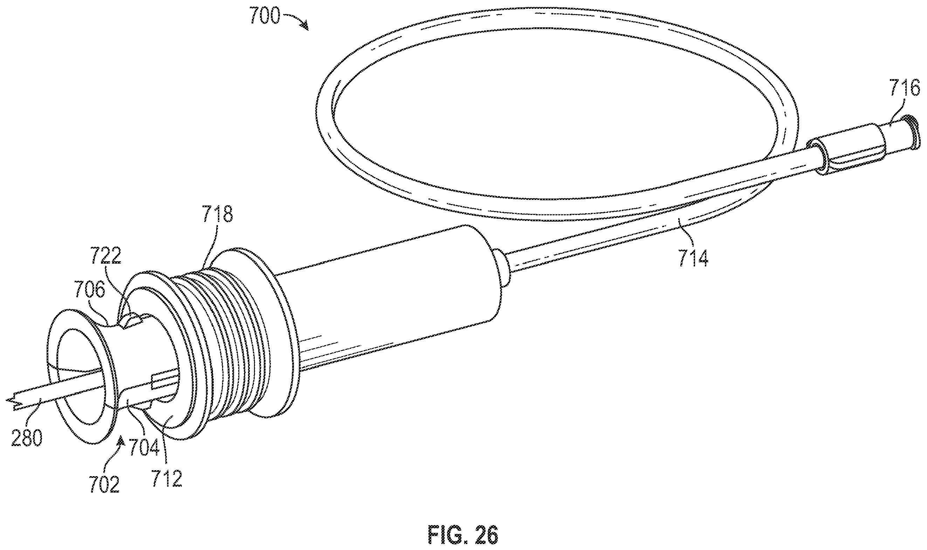

In some examples, aspiration or flushing of a catheter shaft (e.g., the sleeve shaft) can be performed with another sealing mechanism that comprises a clamshell mechanism that creates a seal around the sleeve shaft when closed (FIGS. 25 and 26).

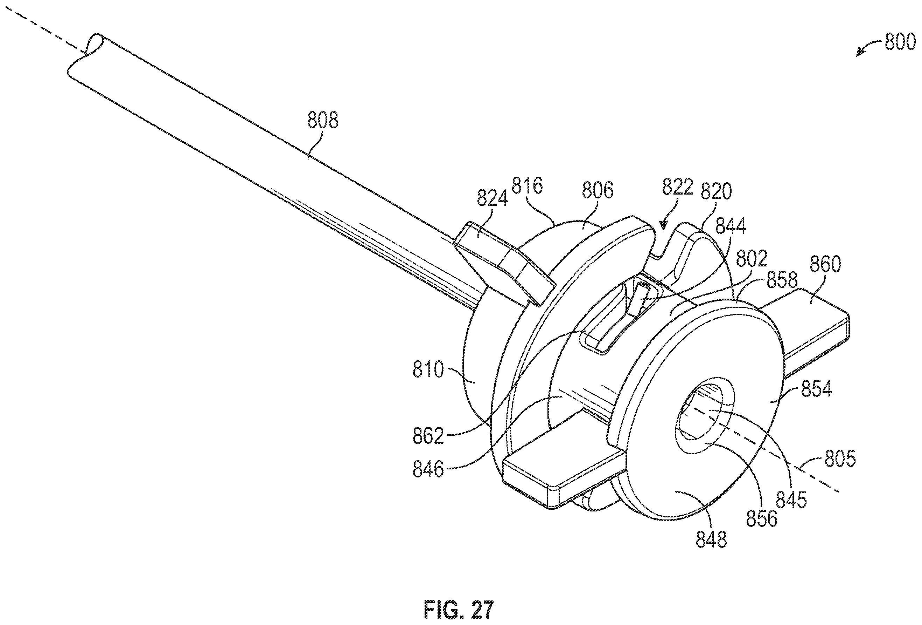

FIGS. 27-34 depict yet a sealing mechanism configured to seal around a catheter shaft (e.g., the sleeve shaft) and allow the catheter shaft to be flushed or aspirated. The sealing mechanism comprises a seal housing containing a seal therein and a locking cap, the locking cap and seal housing configured to rotate relative to one another to move the sealing mechanism into a locked configuration where the seal is radially compressed around the catheter shaft.

### Examples of the Disclosed Technology

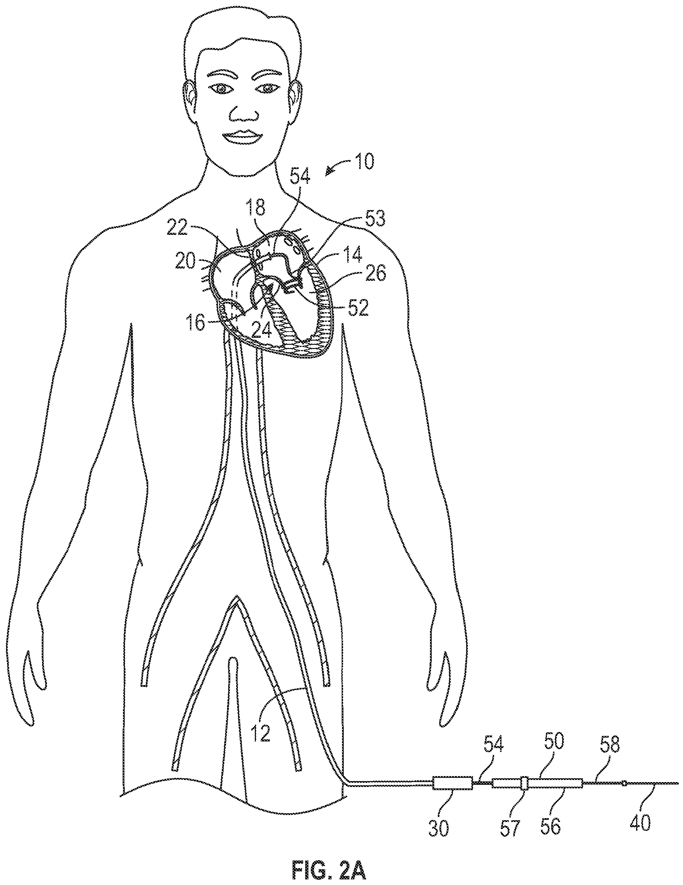

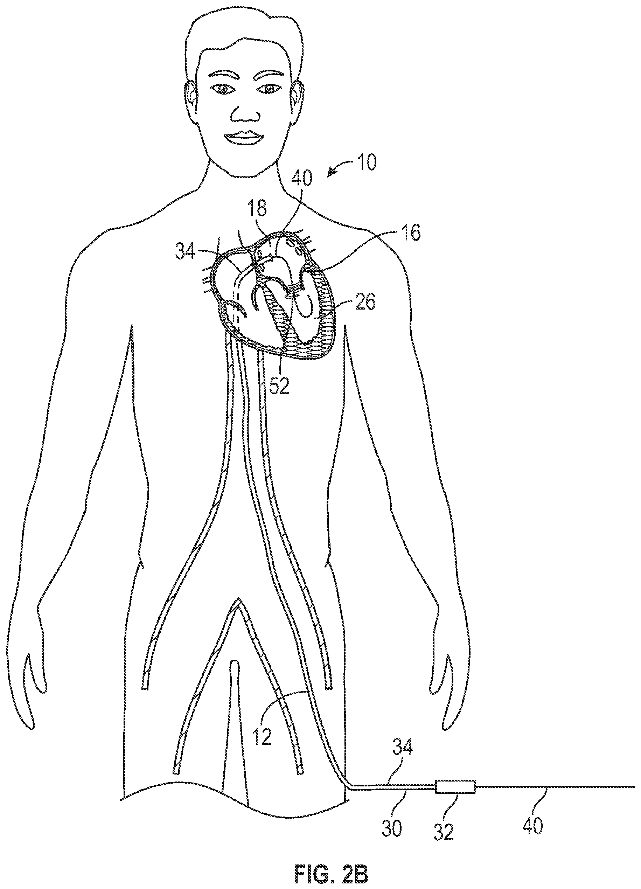

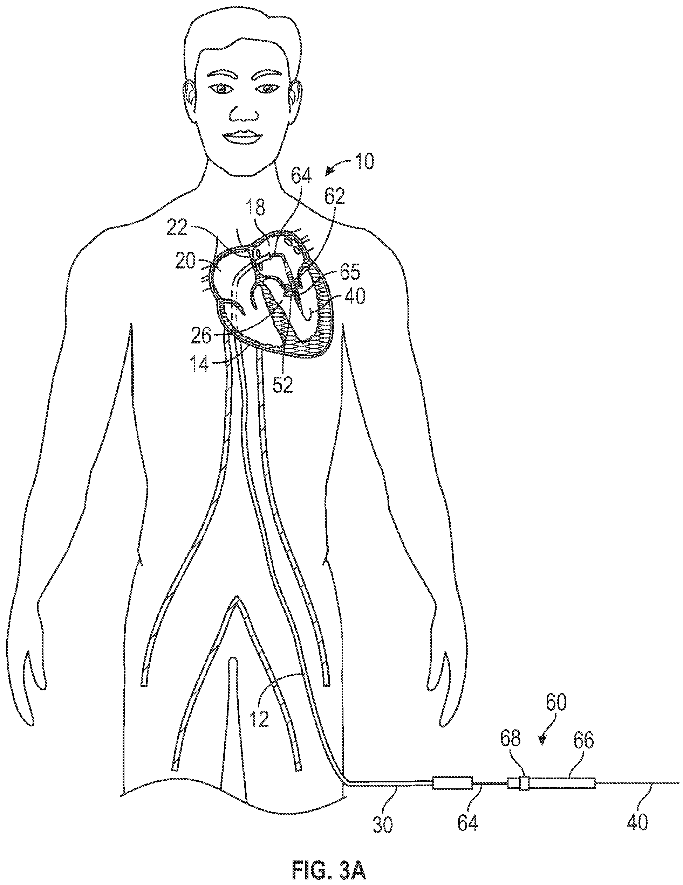

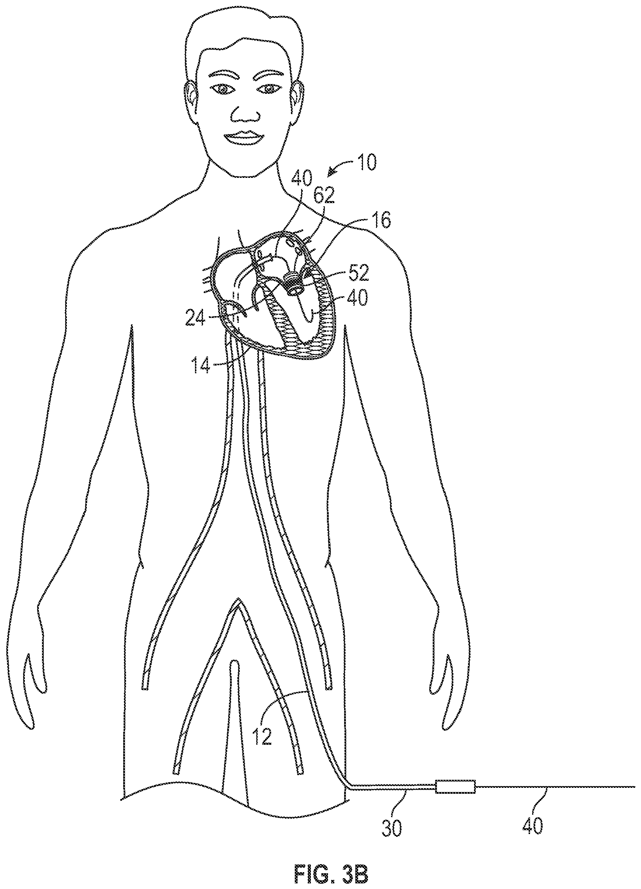

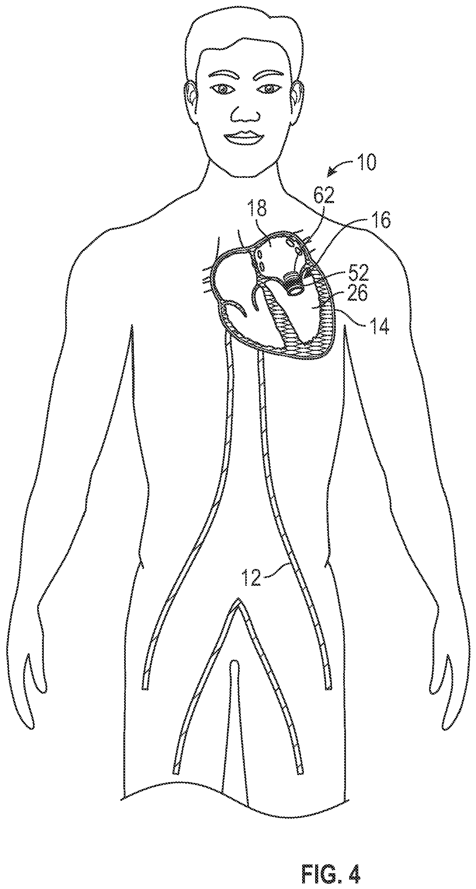

FIGS. 1-4 depict an exemplary transcatheter heart valve replacement procedure (e.g., a mitral valve replacement procedure) which utilizes a docking device 52 and a prosthetic heart valve 62, according to one example. During the procedure, a user first creates a pathway to a patient's native heart valve using a guide catheter 30 (FIG. 1). The user then delivers and implants the docking device 52 at the patient's native heart valve using a docking device delivery apparatus 50 (FIG. 2A) and then removes the docking device delivery apparatus 50 from the patient 10 after implanting the docking device 52 (FIG. 2B). The user then implants the prosthetic heart valve 62 within the implanted docking device 52 using a prosthetic valve delivery apparatus 60 (FIG. 3A). Thereafter, the user removes the prosthetic valve delivery apparatus 60 from the patient 10 (FIG. 3B), as well as the guide catheter 30 (FIG. 4).

FIG. 1 depicts a first stage in a mitral valve replacement procedure, according to one example, where the guide catheter 30 and a guidewire 40 are inserted into a blood vessel 12 of a patient 10 and navigated through the blood vessel 12, into a heart 14 of the patient 10, and toward the native mitral valve 16. Together, the guide catheter 30 and the guidewire 40 can provide a path for the docking device delivery apparatus 50 and the prosthetic valve delivery apparatus 60 to be navigated through and along, to the implantation site (the native mitral valve 16 or native mitral valve annulus).

Initially, the user may first make an incision in the patient's body to access the blood vessel 12. For example, in the example illustrated in FIG. 1, the user may make an incision in the patient's groin to access a femoral vein. Thus, in such examples, the blood vessel 12 may be a femoral vein.

After making the incision at the blood vessel 12, the user may insert the guide catheter 30, the guidewire 40, and/or additional devices (such as an introducer device or transseptal puncture device) through the incision and into the blood vessel 12. The guide catheter 30 (which can also be referred to as an "introducer device", "introducer", or "guide sheath") is configured to facilitate the percutaneous introduction of various implant delivery devices (e.g., the docking device delivery apparatus 50 and the prosthetic valve delivery apparatus 60) into and through the blood vessel 12 and may extend through the blood vessel 12 and into the heart 14 but may stop short of the native mitral valve 16. The guide catheter 30 can comprise a handle 32 and a shaft 34 extending distally from the handle 32. The shaft 34 can extend through the blood vessel 12 and into the heart 14 while the handle 32 remains outside the body of the patient 10 and can be operated by the user in order to manipulate the shaft 34 (FIG. 1).

The guidewire 40 is configured to guide the delivery apparatuses (e.g., the guide catheter 30, the docking device delivery apparatus 50, the prosthetic valve delivery apparatus 60, additional catheters, or the like) and their associated devices (e.g., docking device, prosthetic heart valve, and the like) to the implantation site within the heart 14, and thus may extend all the way through the blood vessel 12 and into a left atrium 18 of the heart 14 (and in some examples, through the native mitral valve 16 and into a left ventricle of the heart 14) (FIG. 1).

In some instances, a transseptal puncture device or catheter can be used to initially access the left atrium 18, prior to inserting the guidewire 40 and the guide catheter 30. For example, after making the incision to the blood vessel 12, the user may insert a transseptal puncture device through the incision and into the blood vessel 12. The user may guide the transseptal puncture device through the blood vessel 12 and into the heart 14 (e.g., through the femoral vein and into the right atrium 20). The user can then make a small incision in an atrial septum 22 of the heart 14 to allow access to the left atrium 18 from the right atrium 20. The user can then insert and advance the guidewire 40 through the transseptal puncture device within the blood vessel 12 and through the incision in the atrial septum 22 into the left atrium 18. Once the guidewire 40 is positioned within the left atrium 18 and/or the left ventricle 26, the transseptal puncture device can be removed from the patient 10. The user can then insert the guide catheter 30 into the blood vessel 12 and advance the guide catheter 30 into the left atrium 18 over the guidewire 40 (FIG. 1).

In some instances, an introducer device can be inserted through a lumen of the guide catheter 30 prior to inserting the guide catheter 30 into the blood vessel 12. In some instances, the introducer device can include a tapered end that extends out a distal tip of the guide catheter 30 and that is configured to guide the guide catheter 30 into the left atrium 18 over the guidewire 40. Additionally, in some instances the introducer device can include a proximal end portion that extends out a proximal end of the guide catheter 30. Once the guide catheter 30 reaches the left atrium 18, the user can remove the introducer device from inside the guide catheter 30 and the patient 10. Thus, only the guide catheter 30 and the guidewire 40 remain inside the patient 10. The guide catheter 30 is then in position to receive an implant delivery apparatus and help guide it to the left atrium 18, as described further below.

FIG. 2A depicts a second stage in the exemplary mitral valve replacement procedure where a docking device 52 is being implanted at the native mitral valve 16 of the heart 14 of the patient 10 using a docking device delivery apparatus 50 (which may also be referred to as an "implant catheter" and/or a "docking device delivery device").

In general, the docking device delivery apparatus 50 comprises a delivery shaft 54, a handle 56, and a pusher assembly 58. The delivery shaft 54 is configured to be advanced through the patient's vasculature (blood vessel 12) and to the implantation site (e.g., native mitral valve 16) by the user and may be configured to retain the docking device 52 in a distal end portion 53 of the delivery shaft 54. In some examples, the distal end portion 53 of the delivery shaft 54 retains the docking device 52 therein in a straightened delivery configuration.

The handle 56 of the docking device delivery apparatus 50 is configured to be gripped and/or otherwise held by the user, outside the body of the patient 10, to advance the delivery shaft 54 through the patient's vasculature (e.g., blood vessel 12).

In some examples, the handle 56 can comprise one or more articulation members 57 (or rotatable knobs) that are configured to aid in navigating the delivery shaft 54 through the blood vessel 12. For example, the one or more articulation members 57 can comprise one or more of knobs, buttons, wheels, and/or other types of physically adjustable control members that are configured to be adjusted by the user to flex, bend, twist, turn, and/or otherwise articulate a distal end portion 53 of the delivery shaft 54 to aid in navigating the delivery shaft 54 through the blood vessel 12 and within the heart 14.

The pusher assembly 58 can be configured to deploy and/or implant the docking device 52 at the implantation site (e.g., the native mitral valve 16). For example, the pusher assembly 58 is configured to be adjusted by the user to push the docking device 52 out of the distal end portion 53 of the delivery shaft 54. A shaft of the pusher assembly 58 can extend through the delivery shaft 54 and can be disposed adjacent to the docking device 52 within the delivery shaft 54. In some examples, the docking device 52 can be releasably coupled to the shaft of the pusher assembly 58 via a connection mechanism of the docking device delivery apparatus 50 such that the docking device 52 can be released after being deployed at the native mitral valve 16.

Further details of the docking device delivery apparatus and its variants are described in International Publication No. WO2020/247907.

Referring again to FIG. 2A, after the guide catheter 30 is positioned within the left atrium 18, the user may insert the docking device delivery apparatus 50 (e.g., the delivery shaft 54) into the patient 10 by advancing the delivery shaft 54 of the docking device delivery apparatus 50 through the guide catheter 30 and over the guidewire 40. In some examples, the guidewire 40 can be at least partially retracted away from the left atrium 18 and into the guide catheter 30. The user may then continue to advance the delivery shaft 54 of the docking device delivery apparatus 50 through the blood vessel 12 along the guidewire 40 until the delivery shaft 54 reaches the left atrium 18, as illustrated in FIG. 2A. Specifically, the user may advance the delivery shaft 54 of the docking device delivery apparatus 50 by gripping and exerting a force on (e.g., pushing) the handle 56 of the docking device delivery apparatus 50 toward the patient 10. While advancing the delivery shaft 54 through the blood vessel 12 and the heart 14, the user may adjust the one or more articulation members 57 of the handle 56 to navigate the various turns, corners, constrictions, and/or other obstacles in the blood vessel 12 and the heart 14.

Once the delivery shaft 54 reaches the left atrium 18 and extends out of a distal end of the guide catheter 30, the user can position the distal end portion 53 of the delivery shaft 54 at and/or near the posteromedial commissure of the native mitral valve 16 using the handle 56 (e.g., the articulation members 57). The user may then push the docking device 52 out of the distal end portion 53 of the delivery shaft 54 with the shaft of the pusher assembly 58 to deploy and/or implant the docking device 52 within the annulus of the native mitral valve 16.

In some examples, the docking device 52 may be constructed from, formed of, and/or comprise a shape memory material, and as such, may return to its original, pre-formed shape when it exits the delivery shaft 54 and is no longer constrained by the delivery shaft 54. As one example, the docking device 52 may originally be formed as a coil, and thus may wrap around leaflets 24 of the native mitral valve 16 as it exits the delivery shaft 54 and returns to its original coiled configuration.

After pushing a ventricular portion of the docking device 52 (e.g., the portion of the docking device 52 shown in FIG. 2A that is configured to be positioned within a left ventricle 26 and/or on the ventricular side of the native mitral valve 16), the user may then deploy the remaining portion of the docking device 52 (e.g., an atrial portion of the docking device 52) from the delivery shaft 54 within the left atrium 18 by retracting the delivery shaft 54 away from the posteromedial commissure of the native mitral valve 16.

After deploying and implanting the docking device 52 at the native mitral valve 16, the user may disconnect the docking device delivery apparatus 50 from the docking device 52. Once the docking device 52 is disconnected from the docking device delivery apparatus 50, the user may retract the docking device delivery apparatus 50 out of the blood vessel 12 and away from the patient 10 so that the user can deliver and implant a prosthetic heart valve 62 within the implanted docking device 52 at the native mitral valve 16.

FIG. 2B depicts this third stage in the mitral valve replacement procedure, where the docking device 52 has been fully deployed and implanted at the native mitral valve 16 and the docking device delivery apparatus 50 (including the delivery shaft 54) has been removed from the patient 10 such that only the guidewire 40 and the guide catheter 30 remain inside the patient 10. In some examples, after removing the docking device delivery apparatus, the guidewire 40 can be advanced out of the guide catheter 30, through the implanted docking device 52 at the native mitral valve 16, and into the left ventricle 26 (FIG. 2A). As such, the guidewire 40 can help to guide the prosthetic valve delivery apparatus 60 through the annulus of the native mitral valve 16 and at least partially into the left ventricle 26.

As illustrated in FIG. 2B, the docking device 52 can comprise a plurality of turns (or coils) that wrap around the leaflets 24 of the native mitral valve 16 (within the left ventricle 26). The implanted docking device 52 has a more cylindrical shape than the annulus of the native mitral valve 16, thereby providing a geometry that more closely matches the shape or profile of the prosthetic heart valve to be implanted. As a result, the docking device 52 can provide a tighter fit, and thus a better seal, between the prosthetic heart valve and the native mitral valve 16, as described further below.

FIG. 3A depicts a fourth stage in the mitral valve replacement procedure where the user is delivering and/or implanting a prosthetic heart valve 62 (which can also be referred to herein as a "transcatheter prosthetic heart valve" or "THV" for short, "replacement heart valve," and/or "prosthetic mitral valve") within the docking device 52 using a prosthetic valve delivery apparatus 60.

As shown in FIG. 3A, the prosthetic valve delivery apparatus 60 can comprise a delivery shaft 64 and a handle 66, the delivery shaft 64 extending distally from the handle 66. The delivery shaft 64 is configured to extend into the patient's vasculature to deliver, implant, expand, and/or otherwise deploy the prosthetic heart valve 62 within the docking device 52 at the native mitral valve 16. The handle 66 is configured to be gripped and/or otherwise held by the user to advance the delivery shaft 64 through the patient's vasculature.

In some examples, the handle 66 can comprise one or more articulation members 68 that are configured to aid in navigating the delivery shaft 64 through the blood vessel 12 and the heart 14. Specifically, the articulation member(s) 68 can comprise one or more of knobs, buttons, wheels, and/or other types of physically adjustable control members that are configured to be adjusted by the user to flex, bend, twist, turn, and/or otherwise articulate a distal end portion of the delivery shaft 64 to aid in navigating the delivery shaft 64 through the blood vessel 12 and into the left atrium 18 and left ventricle 26 of the heart 14.

In some examples, the prosthetic valve delivery apparatus 60 can include an expansion mechanism 65 that is configured to radially expand and deploy the prosthetic heart valve 62 at the implantation site. In some instances, as shown in FIG. 3A, the expansion mechanism 65 can comprise an inflatable balloon that is configured to be inflated to radially expand the prosthetic heart valve 62 within the docking device 52. The inflatable balloon can be coupled to the distal end portion of the delivery shaft 64.

In some examples, the prosthetic heart valve 62 can be self-expanding and can be configured to radially expand on its own upon removable of a sheath or capsule covering the radially compressed prosthetic heart valve 62 on the distal end portion of the delivery shaft 64. In some examples, the prosthetic heart valve 62 can be mechanically expandable and the prosthetic valve delivery apparatus 60 can include one or more mechanical actuators (e.g., the expansion mechanism) configured to radially expand the prosthetic heart valve 62.

As shown in FIG. 3A, the prosthetic heart valve 62 is mounted around the expansion mechanism 65 (the inflatable balloon) on the distal end portion of the delivery shaft 64, in a radially compressed configuration.

To navigate the distal end portion of the delivery shaft 64 to the implantation site, the user can insert the prosthetic valve delivery apparatus 60 (the delivery shaft 64) into the patient 10 through the guide catheter 30 and over the guidewire 40. The user can continue to advance the prosthetic valve delivery apparatus 60 along the guidewire 40 (through the blood vessel 12) until the distal end portion of the delivery shaft 64 reaches the native mitral valve 16, as illustrated in FIG. 3A. More specifically, the user can advance the delivery shaft 64 of the prosthetic valve delivery apparatus 60 by gripping and exerting a force on (e.g., pushing) the handle 66. While advancing the delivery shaft 64 through the blood vessel 12 and the heart 14, the user can adjust the one or more articulation members 68 of the handle 66 to navigate the various turns, corners, constrictions, and/or other obstacles in the blood vessel 12 and heart 14.

The user can advance the delivery shaft 64 along the guidewire 40 until the radially compressed prosthetic heart valve 62 mounted around the distal end portion of the delivery shaft 64 is positioned within the docking device 52 and the native mitral valve 16. In some examples, as shown in FIG. 3A, a distal end of the delivery shaft 64 and a least a portion of the radially compressed prosthetic heart valve 62 can be positioned within the left ventricle 26.

Once the radially compressed prosthetic heart valve 62 is appropriately positioned within the docking device 52 (FIG. 3A), the user can manipulate one or more actuation mechanisms of the handle 66 of the prosthetic valve delivery apparatus 60 to actuate the expansion mechanism 65 (e.g., inflate the inflatable balloon), thereby radially expanding the prosthetic heart valve 62 within the docking device 52.

FIG. 3B shows a fifth stage in the mitral valve replacement procedure where the prosthetic heart valve 62 in its radially expanded configuration and implanted within the docking device 52 in the native mitral valve 16. As shown in FIG. 3B, the prosthetic heart valve 62 is received and retained within the docking device 52. Thus, the docking device 52 aids in anchoring the prosthetic heart valve 62 within the native mitral valve 16. The docking device 52 can enable better sealing between the prosthetic heart valve 62 and the leaflets 24 of the native mitral valve 16 to reduce paravalvular leakage around the prosthetic heart valve 62.

As also shown in FIG. 3B, after the prosthetic heart valve 62 has been fully deployed and implanted within the docking device 52 at the native mitral valve 16, the prosthetic valve delivery apparatus 60 (including the delivery shaft 64) is removed from the patient 10 such that only the guidewire 40 and the guide catheter 30 remain inside the patient 10.

FIG. 4 depicts a sixth stage in the mitral valve replacement procedure, where the guidewire 40 and the guide catheter 30 have been removed from the patient 10.

Although FIGS. 1-4 specifically depict a mitral valve replacement procedure, it should be appreciated that the same and/or similar procedure may be utilized to replace other heart valves (e.g., tricuspid, pulmonary, and/or aortic valves). Further, the same and/or similar delivery apparatuses (e.g., docking device delivery apparatus 50, prosthetic valve delivery apparatus 60, guide catheter 30, and/or guidewire 40), docking devices (e.g., docking device 52), replacement heart valves (e.g., prosthetic heart valve 62), and/or components thereof may be utilized for replacing these other heart valves.

For example, when replacing a native tricuspid valve, the user may also access the right atrium 20 via a femoral vein but may not need to cross the atrial septum 22 into the left atrium 18. Instead, the user may leave the guidewire 40 in the right atrium 20 and perform the same and/or similar docking device implantation process at the tricuspid valve. Specifically, the user may push the docking device 52 out of the delivery shaft 54 around the ventricular side of the tricuspid valve leaflets, release the remaining portion of the docking device 52 from the delivery shaft 54 within the right atrium 20, and then remove the delivery shaft 54 of the docking device delivery apparatus 50 from the patient 10. The user may then advance the guidewire 40 through the tricuspid valve into the right ventricle and perform the same and/or similar prosthetic heart valve implantation process at the tricuspid valve, within the docking device 52. Specifically, the user may advance the delivery shaft 64 of the prosthetic valve delivery apparatus 60 through the patient's vasculature along the guidewire 40 until the prosthetic heart valve 62 is positioned/disposed within the docking device 52 and the tricuspid valve. The user may then expand the prosthetic heart valve 62 within the docking device 52 before removing the prosthetic valve delivery apparatus 60 from the patient 10. In some examples, the user may perform the same and/or similar process to replace the aortic valve but may access the aortic valve from the outflow side of the aortic valve via a femoral artery.

Further, although FIGS. 1-4 depict a mitral valve replacement procedure that accesses the native mitral valve 16 from the left atrium 18 via the right atrium 20 and femoral vein, it should be appreciated that the native mitral valve 16 may alternatively be accessed from the left ventricle 26. For example, the user may access the native mitral valve 16 from the left ventricle 26 via the aortic valve by advancing one or more delivery apparatuses through an artery to the aortic valve, and then through the aortic valve into the left ventricle 26.

FIG. 5 shows an example of a docking device 100 configured to receive a prosthetic heart valve. For example, the docking device 100 can be implanted within a native valve annulus, as described above with reference to FIGS. 1-2B. The docking device 100 can be used in lieu of docking device 52 in FIGS. 2A-4, and as such, the docking device 100 can be configured to receive and secure a prosthetic valve within the docking device, thereby securing the prosthetic valve at the native valve annulus.

Referring to FIG. 5, the docking device 100 can comprise two main components: a coil 102 and a guard member 104 covering at least a portion of the coil 102. In certain examples, the coil 102 can include a shape memory material (e.g., Nitinol) such that the docking device 100 (and the coil 102) can move from a substantially straight configuration (also referred to as "delivery configuration") when disposed within a delivery sleeve (e.g., sleeve shaft) of a delivery apparatus (as described more fully below) to a helical configuration (also referred to as "deployed configuration," as shown in FIG. 5) after being removed from the delivery sleeve (e.g., sleeve shaft).

The coil 102 has a proximal end 102p and a distal end 102d. When being disposed within the delivery sleeve (e.g., during delivery of the docking device into the vasculature of a patient), a body of the coil 102 between the proximal end 102p and distal end 102d can form a generally straight delivery configuration (e.g., without any coiled or looped portions) so as to maintain a small radial profile when moving through a patient's vasculature. After being removed from the delivery sleeve and deployed at an implant position, the coil 102 can move from the delivery configuration to the helical deployed configuration and wrap around native tissue adjacent the implant position. For example, when implanting the docking device at the location of a native valve, the coil 102 can be configured to surround native leaflets of the native valve (and the chordae tendineae that connects native leaflets to adjacent papillary muscles, if present).

The docking device 100 can be releasably coupled to a delivery apparatus. In certain examples, the docking device 100 can be coupled to a delivery apparatus via a release suture that can be configured to be tied to the docking device 100 and cut for removal (as described further below with reference to FIGS. 6 and 8). In one example, the release suture can be tied to the docking device 100 through an eyelet or eyehole located adjacent the proximal end 102p of the coil. In some examples, the release suture can be tied around a circumferential recess that is located adjacent the proximal end 102p of the coil 102.

In some examples, the docking device 100 in the deployed configuration can be configured to fit at the mitral valve position. In some examples, the docking device can also be shaped and/or adapted for implantation at other native valve positions as well, such as at the tricuspid valve. In some examples, the geometry of the docking device 100 can be configured to engage the native anatomy, which can, for example, provide for increased stability and reduction of relative motion between the docking device 100, the prosthetic valve docked therein, and/or the native anatomy.

As shown in FIG. 5, the coil 102 in the deployed configuration can include a leading turn 106 (or "leading coil"), a central region 108, and stabilization turn 110 (or "stabilization coil"). The central region 108 can possess one or more helical turns having substantially equal inner diameters. The leading turn 106 can extend from a distal end of the central region 108 and has a diameter greater than the diameter of the central region 108 (in one or more configurations). The stabilization turn 110 can extend from a proximal end of the central region 108 and has a diameter greater than the diameter of the central region 108 (in one or more configurations).

In certain examples, the central region 108 can include a plurality of helical turns, such as a proximal turn 108p in connection with the stabilization turn 110, a distal turn 108d in connection with the leading turn 106, and one or more intermediate turns 108m disposed between the proximal turn 108p and the distal turn 108d. In the example shown in FIG. 5, there is only one intermediate turn 108m between the proximal turn 108p and the distal turn 108d.

In some examples, there can be more than one intermediate turn 108m (e.g., two, three, or the like) between the proximal turn 108p and the distal turn 108d. Some of the helical turns in the central region 108 can be full turns (i.e., rotating 360 degrees). In some instances, the proximal turn 108p and/or the distal turn 108d can be partial turns (e.g., rotating less than 360 degrees, such as 180 degrees, 270 degrees, etc.).

A size of the docking device 100 can be generally selected based on the size of the desired prosthetic valve to be implanted in the patient. In certain examples, the central region 108 can be configured to retain a radially expandable prosthetic valve. For example, the inner diameter of the helical turns in the central region 108 can be configured to be smaller than an outer diameter of the prosthetic valve when the prosthetic valve is radially expanded so that additional radial tension can act between the central region 108 and the prosthetic valve to hold the prosthetic valve in place. The helical turns (e.g., 108p, 108m, 108d) in the central region 108 are also referred to herein as "functional turns."

The stabilization turn 110 can be configured to help stabilize the docking device 100 in the desired position within the surrounding anatomy at the implantation site. For example, the radial dimension of the stabilization turn 110 can be significantly larger than the radial dimension of the coil in the central region 108, so that the stabilization turn 110 can flare or extend sufficiently outwardly so as to abut or push against the walls of the atrium of the heart, thereby improving the ability of the docking device 100 to stay in its desired position prior to the implantation of the prosthetic valve. In some examples, the diameter of stabilization turn 110 is larger than the native valve annulus, native valve plane, and atrium for better stabilization. In some examples, the stabilization turn 110 can be a full turn (i.e., rotating about 360 degrees). In some examples, the stabilization turn 110 can be a partial turn (e.g., rotating between about 180 degrees and about 270 degrees).

In one particular example, when implanting the docking device 100 at the native mitral valve location, the functional turns in the central region 108 can be disposed predominantly in the left ventricle and the stabilization turn 110 can be disposed predominantly in the left atrium. The stabilization turn 110 can be configured to provide one or more points or regions of contact between the docking device 100 and the left atrial wall, such as at least three points of contact in the left atrium or complete contact on the left atrial wall. In certain examples, the points of contact between the docking device 100 and the left atrial wall can form a plane that is approximately parallel to a plane of the native mitral valve.

As noted above, the leading turn 106 can have a larger radial dimension than the helical turns in the central region 108. The leading turn 106 can help more easily guide the coil 102 around and/or through the chordae tendineae geometry and adequately around all native leaflets of the native valve (e.g., the native mitral valve, tricuspid valve, etc.). For example, once the leading turn 106 is navigated around the desired native anatomy, the remaining coil (such as the functional turns) of the docking device 100 can also be guided around the same features. In some examples, the leading turn 106 can be a full turn (i.e., rotating about 360 degrees). In some examples, the leading turn 106 can be a partial turn (e.g., rotating between about 180 degrees and about 270 degrees). In some examples, when a prosthetic valve is radially expanded within the central region 108 of the coil, the functional turns in the central region 108 can be further radially expanded. As a result, the leading turn 106 can be pulled in the proximal direction, can decrease in diameter, and can become a part of the functional turns in the central region 108.

In certain examples, at least a portion of the coil 102 can be surrounded by a first cover. The first cover can be constructed of various native and/or synthetic materials. In one particular example, the first cover can include expanded polytetrafluoroethylene (ePTFE). In certain examples, the first cover is configured to be fixedly attached to the coil 102 (e.g., by means of textured surface resistance, suture, glue, thermal bonding, or any other means) so that relative axial movement between the first cover and the coil 102 is restricted or prohibited.

The guard member 104 can constitute a part of a cover assembly for the docking device 100. In some examples, the cover assembly can also include the first cover.

In a typical example as shown in FIG. 5, when the docking device 100 is in the deployed configuration, the guard member 104 can be configured to cover a portion of the stabilization turn 110 of the coil 102. In certain examples, the guard member 104 can be configured to cover at least a portion of the central region 108 of the coil 102, such as a portion of the proximal turn 108p. In certain examples, the guard member 104 can extend over the entirety of the coil 102.

In some examples, the guard member 104 can radially expand so as to help prevent and/or reduce paravalvular leakage. Specifically, the guard member 104 can be configured to radially expand such that an improved seal is formed closer to and/or against a prosthetic valve deployed within the docking device 100. In some examples, the guard member 104 can be configured to prevent and/or inhibit leakage at the location where the docking device 100 crosses between leaflets of the native valve (e.g., at the commissures of the native leaflets).

In some examples, when the docking device 100 is deployed at a native atrioventricular valve (e.g., mitral valve or tricuspid valve) and the guard member 104 covers predominantly a portion of the stabilization turn 110 and/or a portion of the central region 108, the guard member 104 can help cover an atrial side of an atrioventricular valve to prevent and/or inhibit blood from leaking through the native leaflets, commissures, and/or around an outside of the prosthetic valve by blocking blood in the atrium from flowing in an atrial to ventricular direction (i.e., antegrade blood flow)-other than through the prosthetic valve.

In some examples, the guard member 104 can be positioned on a ventricular side of an atrioventricular valve to prevent and/or inhibit blood from leaking through the native leaflets, commissures, and/or around an outside of the prosthetic valve by blocking blood in the ventricle from flowing in a ventricular to atrial direction (i.e., retrograde blood flow).

In some examples, a distal end portion 104d of the guard member 104 can be fixedly coupled to the coil 102 (e.g., via a distal suture), and a proximal end portion 104p of the guard member 104 can be axially moveable relative to the coil 102.

In some instances, when the guard member 104 is in the radially expanded state, the proximal end portion 104p of the guard member 104 can have a tapered shape as shown in FIG. 5, such that the diameter of the proximal end portion 104p gradually increases from a proximal terminal end of the guard member 104 to a distally located body portion of the guard member 104. This can, for example, help to facilitate loading the docking device into a delivery sleeve (e.g., sleeve shaft) of the delivery apparatus and/or retrieval and/or repositioning of the docking device into the delivery apparatus during an implantation procedure.

FIGS. 6-10 illustrate examples of a delivery apparatus (which can also be referred to as a delivery system) 200 configured to deliver a docking device (such as docking device 100 described above with reference to FIG. 5) to a target implantation site (e.g., a heart and/or native valve of an animal, human, cadaver, cadaver heart, anthropomorphic ghost, and/or the like). In some examples, the delivery apparatus 200 can be a transcatheter delivery apparatus that can be used to guide a docking device mounted therein through a patient's vasculature, as explained above with reference to FIGS. 1-2B.

The exemplary delivery apparatus 200 is shown in FIG. 6 with a docking device 232 at least partially deployed from a distal end of the delivery apparatus 200 (e.g., for illustration purposes). In some examples, the docking device 232 can be the docking device 100 described above with reference to FIG. 5. FIGS. 7 and 8 show a distal end portion of the delivery apparatus 200 with the docking device 232 deployed from an outer shaft 260 of the delivery apparatus with a sleeve shaft 280 covering the docking device 232 (FIG. 7) and after the sleeve shaft 280 has been removed from the docking device 232 (but prior to disconnecting the docking device 232 from the delivery apparatus 200) (FIG. 8). FIGS. 9 and 10 are schematic cross-sectional views of the delivery apparatus 200 showing multiple lumens formed between the coaxial components of the delivery apparatus 200.

Returning to FIG. 6, the delivery apparatus 200 can include a handle assembly 220 and an outer shaft (e.g., delivery catheter) 260 extending distally from the handle assembly 220. The handle assembly 220 can include a handle 222 and a hub assembly 230 extending from a proximal end of the handle 222. As shown in FIG. 6, the handle assembly 220 can include a handle 222 including one or more knobs, buttons, wheels, or the like. For example, as shown in FIG. 6, the handle 222 can include knobs 224 and 226 which can be configured to control flexing of the delivery apparatus (e.g., the outer shaft 260). The outer shaft 260 extends distally from the handle 222 while the hub assembly 230 extends proximally from the handle 222.

The delivery apparatus 200 can include a pusher shaft 290 (FIGS. 6 and 8-10) and a sleeve shaft 280 (FIGS. 7-10) which are coaxially located within the outer shaft 260 (FIGS. 9 and 10) and each have portions that extend into the handle assembly 220. The pusher shaft 290 can be configured to deploy the docking device 232 from inside a distal end portion of the outer shaft 260, upon reaching the target implantation site, and the sleeve shaft 280 can be configured to cover the docking device 232 while inside the delivery apparatus 200 (FIGS. 9 and 10) and while being positioned at the target implantation site (FIG. 7). Further, the delivery apparatus 200 can be configured to adjust an axial position of the sleeve shaft 280 to remove a sleeve portion (e.g., distal section) of the sleeve shaft 280 from the docking device 232, after implantation at the target implantation site (FIG. 8). FIGS. 7 and 8 are perspective views showing the exemplary docking device 232 deployed from the outer shaft 260 of the delivery apparatus 200, covered by the distal (or sleeve) portion 282 of the sleeve shaft 280 (FIG. 7), and the exemplary docking device 232 after the sleeve shaft 280 has been retracted back into the outer shaft 260 (FIG. 8).

Thus, the sleeve shaft 270 can be removable from the docking device 232. In some examples, the distal portion 282 of the sleeve shaft 280 can have an outer surface comprising a lubricious or low-friction material that makes it easier to slide the docking device 232 into place with the native anatomy at the implantation site.

As shown in FIGS. 6 and 8, during delivery, the docking device 232 can be coupled to the delivery apparatus 200 via a release suture 236 (or other retrieval line comprising a string, yarn, or other material that can be configured to be tied around the docking device and cut for removal) that can extend through the pusher shaft 290. The release suture 236 can extend through the delivery apparatus 200, through an inner lumen of the pusher shaft 290, to a suture lock assembly 206 of the delivery apparatus 200.

As shown in FIG. 6, the hub assembly 230 can include the suture lock assembly (e.g., suture lock) 206 and a sleeve handle 234 attached thereto. The hub assembly 230 can be configured to control the pusher shaft 290 and the sleeve shaft 280 of the delivery apparatus 200, together (e.g., move them axially together), while the sleeve handle 234 can control an axial position of the sleeve shaft 280 relative to the pusher shaft 290. In this way, operation of the various components of the handle assembly 220 can actuate and control operation of the components arranged within the outer shaft 260. In some examples, as shown in FIG. 6, the hub assembly 230 can be coupled to the handle 222 via a connector 240.

In some examples, the hub assembly 230 can comprise a Y-shaped connector (e.g., adaptor) having a straight section (e.g., straight conduit) 202 and at least one branch (e.g., branch conduit) 204 (though, in some examples, it can include more than one branch) (FIG. 6). In some examples, the suture lock assembly 206 can be attached to the branch 204 and the sleeve handle 234 (e.g., sleeve actuating handle) can be arranged at a proximal end of the straight section 202.

Further details on the delivery apparatus 200 and its variants, including details on a suture lock assembly and a pusher shaft and sleeve shaft assembly for a delivery apparatus for a docking device are described in International Patent Publication No. WO 2020/247907. Further details on additional delivery systems and apparatuses that are configured to deliver a docking device to a target implantation site can be found in U.S. Patent Publication Nos. US2018/0318079, US2018/0263764, and US2018/0177594.

Returning to FIG. 6, the handle assembly 220 can further include one or more flushing ports to supply flush fluid to one or more lumens arranged within the delivery apparatus 200 (e.g., annular lumens arranged between coaxial components of the delivery apparatus 200) in order to reduce potential thrombus formation and/or to de-air components of the delivery apparatus 200 prior to insertion into a patient. FIG. 6 depicts one example where the delivery apparatus 200 includes three flushing ports (e.g., flushing ports 210, 216, and 218). In alternate examples, the delivery apparatus 200 may not include the flushing port 216 or the flushing port 210 can be alternatively placed at an end of the suture lock assembly 206 (e.g., as shown in FIGS. 9 and 10).

For example, as shown in FIGS. 9 and 10 which are exemplary, simplified schematics of the delivery apparatus 200, multiple lumens are formed between the docking device 232, the pusher shaft 290, the sleeve shaft 280, and the outer shaft 260 which are configured to receive fluid. More specifically, a first, pusher shaft lumen 201 can be formed within an interior of the pusher shaft 290 (e.g., within an interior of a main tube 292 of the pusher shaft 290). A second, sleeve shaft lumen 211 is formed within the sleeve shaft 280. Additionally, a third, delivery shaft lumen 215 (or outer shaft lumen) can be formed in an annular space formed between an inner surface of the outer shaft 260 and an outer surface of the sleeve shaft 280.

As shown in FIG. 9, the pusher shaft lumen 201 can receive fluid directly from a first fluid source or flushing port 210, which may be fluidly coupled to a portion of the handle assembly, such as to an end of the suture lock assembly 206, as shown in FIGS. 9 and 10. Alternatively, as shown in FIG. 6, the flushing port 210 can be coupled to a location along the branch 204. A flush fluid flow 203 from the flushing port 210 can travel through the pusher shaft lumen 201, along a length of the main tube 292 of the pusher shaft 290, to a distal end 293 of the pusher shaft 290. A first portion of the flush fluid flow 203 can flow into a first portion 205 of the sleeve shaft lumen 211, which is arranged between an outer surface of the docking device 232 and an inner surface of the distal portion 282 of the sleeve shaft 280, as flush fluid flow 207. In some examples, the flush fluid flow 207 can flow through a guard member 231 of the docking device 232 (which may be the same or similar to guard member 104 of FIG. 5). A second portion of the flush fluid flow 203 can also flow into a second portion 209 of the sleeve shaft lumen 211, which is arranged between an outer surface of the pusher shaft 290 and an inner surface of the sleeve shaft 280, as flush fluid flow 213. The flush fluid flow 213 can continue through the second portion 209 of the sleeve shaft lumen 211 and into a shell portion 294 of the pusher shaft. Since the delivery shaft lumen 215 is fluidly coupled with the shell portion 294, the flush fluid flow 213 can continue into and through the delivery shaft lumen 215, toward a distal end 262 of the outer shaft 260.

As shown in FIG. 10, the lumens of the delivery apparatus 200 can also receive fluid from a second fluid source or flushing port 216. The flushing port 216 can be fluidly coupled to a cavity 254 that is disposed around the main tube 292 of the pusher shaft 290 in the hub assembly 230. The cavity 254 fluidly couples to an annular cavity 219 defined by the shell portion 294, and the annular cavity 219 fluidly couples to the delivery shaft lumen 215. Thus, a flush fluid flow 221 from the flushing port 216 can travel through the cavity 254 and into and through the annular cavity 219. The flush fluid flow 221 can then split into a first flush fluid flow 217 into and through the delivery shaft lumen 215 and a second flush fluid flow 223 into and through the sleeve shaft lumen 211.

Although fluid flow can be provided to the sleeve shaft lumen 211 in various instances, as described above with reference to FIGS. 9 and 10, since the flush fluid flow may be split between the sleeve shaft lumen 211 and the delivery shaft lumen 215, a threshold fluid pressure for adequately flushing and de-airing the docking device (e.g., the guard member 231 of the docking device 232) may not be reached. Thus, it is desirable to force all, or a majority of the flush fluid flow provided by one or more flushing ports of the delivery apparatus 200 through the sleeve shaft lumen 211 in order to de-air the sleeve shaft lumen 211 and guard member (or alternate covering) of the docking device.

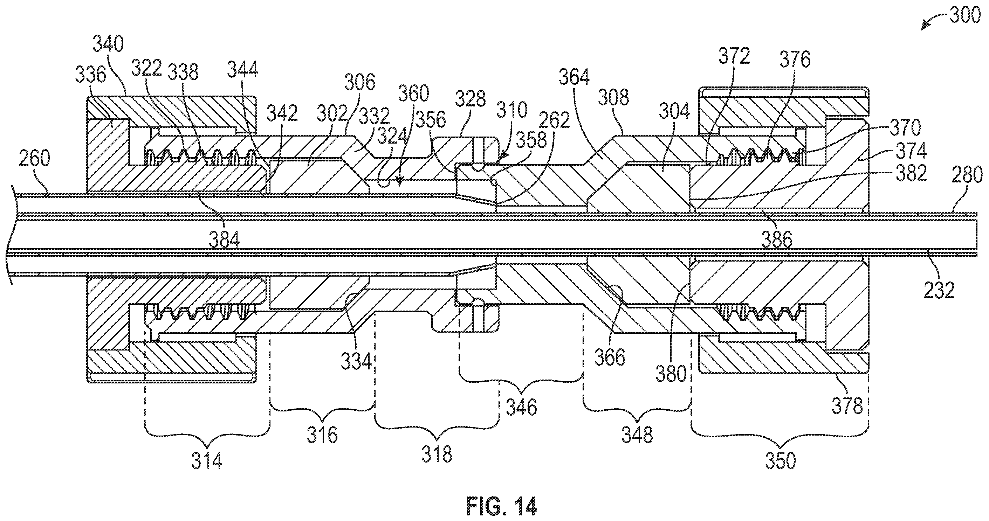

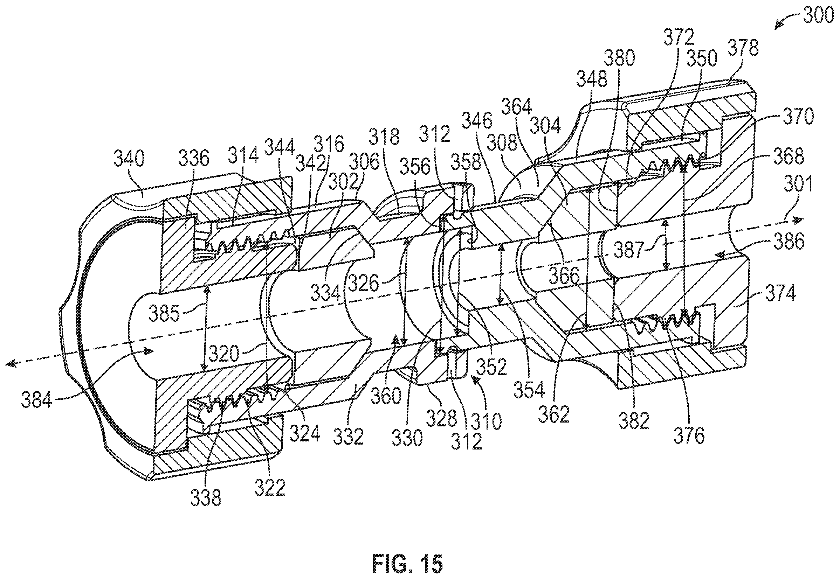

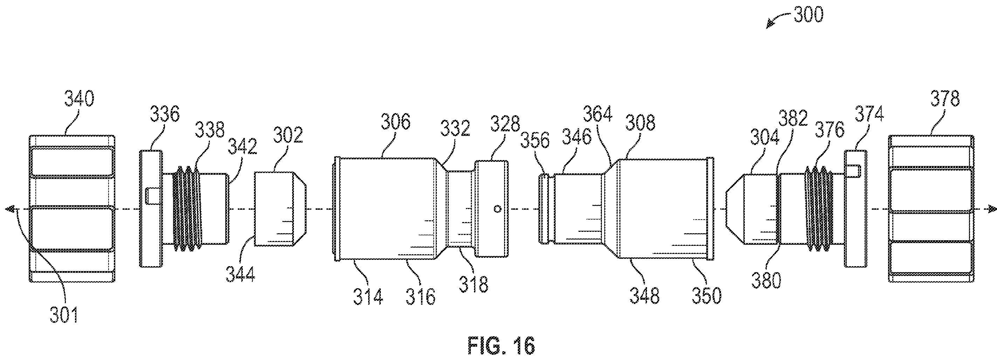

Turning now to FIGS. 11-16, an exemplary sealing mechanism 300 for a catheter that is configured to regulate fluid flow through two shafts of the catheter is shown. For example, the sealing mechanism is configured to seal around two shafts of the catheter that are concentric with one another (or one disposed around the other but having slightly offset central axes) along at least a distal end portion of the catheter and divert fluid flow provided to the catheter through one shaft of the two shafts by blocking fluid flow out an end of another shaft of the two shafts. In some examples, the catheter is a delivery apparatus for an implantable medical device, such as the delivery apparatus 200 of FIGS. 6-10. For example, FIG. 11 (side view) and FIG. 14 (cross-sectional side view) show the sealing mechanism 300 coupled to the outer shaft 260 and the sleeve shaft 280 of the delivery apparatus 200. However, in alternate examples, the sealing mechanism 300 can be used with a variety of catheters and delivery apparatuses including two or more shafts (e.g., inner and outer shafts) with fluidly coupled lumens. FIGS. 12 and 13 show alternate end views of the sealing mechanism 300, FIG. 15 shows a cross-sectional perspective view of the sealing mechanism 300, and FIG. 16 shows an exploded view of the sealing mechanism 300.

The sealing mechanism 300 includes a first seal 302 and a second seal 304 disposed within a housing of the sealing mechanism 300. The housing includes a first seal housing 306 containing the first seal 302 therein and a second seal housing 308 containing the second seal 304 therein. The first seal 302 and the second seal 304 can be annular with an aperture (e.g., central aperture) configured to receive a shaft therethrough, as shown in FIGS. 14 and 15.

The first seal housing 306 and the second seal housing 308 can be coupled to one another at an interface 310 (FIGS. 11, 14, and 15). In some examples, the interface 310 is an overlapping interface where a portion of the first seal housing 306 overlaps a portion of the second seal housing 308 (as shown in FIGS. 11 and 14-16). In alternate examples, the interface 310 is an overlapping interface where a portion of the second seal housing 308 overlaps a portion of the first seal housing 306. In some instances, the first seal housing 306 and the second seal housing 308 can be coupled together by one or more fasteners extending through one or more bores 312 (or apertures) in the first seal housing 306 and the second seal housing 308 (FIGS. 11 and 15).

The first seal housing 306 can comprise a proximal portion 314, an intermediate portion 316, and a distal portion 318 (FIGS. 14-16). The proximal portion 314 has a first inner diameter 320 and includes a plurality of internal threads 322 in an inner surface 324 of the first seal housing 306 (FIG. 15). In some examples, as shown in FIGS. 14 and 15, the first seal 302 can be disposed within the intermediate portion 316 of the first seal housing 306. The intermediate portion 316 can also have the first inner diameter 320. In alternate examples, the first seal 302 can be disposed in a more distal portion of the first seal housing 306.

The distal portion 318 of the first seal housing 306 can have a second inner diameter 326 that is smaller than the first inner diameter 320 (FIG. 15). In some examples, the distal portion 318 can also include an outer collar portion 328 that is configured to receive the second sealing housing 308 therein, at the interface 310 (FIGS. 11 and 14-16). In some instances, the collar portion 328 can have a third inner diameter 330 that is larger than the second inner diameter 326. In some examples, the third inner diameter 330 can be the same as the first inner diameter 320. In alternate examples, the third inner diameter 330 can be larger or smaller than the first inner diameter 320, while still being larger than the second inner diameter 326.

In some examples, the first seal housing 306 can also include a transition portion 332 that includes a taper or angled step 334 that more gradually decreases in diameter from the first inner diameter 320 to the second inner diameter 326. Further, in some instances, the angled step 334 can be annular and extend around the circumference of the first seal housing 306. In alternate examples, instead of being angled, the step of the transition portion 332 can be a right-angled step.

In some instances, the first seal 302 is shaped such that its distal end portion tapers to match the taper or angling of the angled step 334. As such, the first seal 302 can be shaped to fit within the intermediate portion 316 and the transition portion 332, against the angled step 334.

The sealing mechanism 300 can also include a first threaded member 336 coupled to the proximal portion 314 of the first seal housing 306 (FIGS. 14-16). In particular, the first threaded member 336 can include external threads 338 that are configured to mate with the internal threads 322 of the first seal housing 306 (FIGS. 14 and 15). A first knob 340 (or alternative rotatable element) can be fixed to the first threaded member 336 and configured to rotate (FIGS. 11-16). In some instances, the first knob can be coupled or fixed to a proximal end of the first threaded member 336 and disposed around the proximal portion 314 of the first seal housing 306. Rotation of the first knob 340 can cause the first threaded member 336 to rotate relative to the first seal housing 306, thereby causing the first threaded member 336 to travel in an axial direction (relative to a central longitudinal axis 301 of the sealing mechanism 300). As the first threaded member 336 travels distally (toward the second seal housing 308), a distal end 342 of the first threaded member 336 can contact and push against a proximal end 344 of the first seal 302 (FIGS. 14 and 15), thereby compressing the first seal 302 around a shaft disposed therein (e.g., the outer shaft 260 shown in FIG. 14). In this way, the first seal 302 can be tightened around and sealed against a shaft disposed therein by rotating the first knob 340 (and consequently the first threaded member 336). Additionally, compressing the first seal 302 with the first knob 340 can also axially lock the sealing mechanism 300 to the shaft disposed therein, thereby ensuring the sealing mechanism 300 stays connected to the shaft during flushing at relatively high fluid pressures, as described below.

The second seal housing 308 can comprise a proximal portion 346, an intermediate portion 348, and a distal portion 350 (FIGS. 14-16). The proximal portion 346 can have a fourth inner diameter 352 at its proximal end 356 and a fifth inner diameter 354 in a more distal region of the proximal portion 346, where the fifth inner diameter 354 is smaller than the fourth inner diameter 352 (FIG. 15). The proximal end 356 of the proximal portion 346 can interface with and couple to the first seal housing 306, such as to the collar portion 328 (FIGS. 14 and 15). In some instances, the fourth inner diameter 352 can be the same as the second inner diameter 326 of the distal portion 318 of the first seal housing 306.

In some examples, a step 358 in the proximal portion 346 transitions between the fourth inner diameter 352 and the fifth inner diameter 354 (FIGS. 14 and 15). The step 358 can also serve as a stop that is configured to interface with a distal end of a shaft extending through the first seal housing 306 (e.g., the outer shaft 260). For example, as shown in FIG. 14, the distal end 262 of the outer shaft 260 can hit the step 358, which prevents the outer shaft 260 from moving further in the distal direction through the second seal housing 308. A cavity 360 can be defined within the first seal housing 306 and the second seal housing 308, between the first seal 302 and the second seal 304. As shown in FIG. 14, the distal end 262 of the outer shaft 260 can reside in the cavity 360. Further, as explained in more detail below, the cavity 360 can be fluidly sealed by the walls of the first seal housing 306, the second seal housing 308, the first seal 302, and the second seal 304 when the first seal 302 is tightened around a first shaft (e.g., the outer shaft 260) and the second seal 304 is tightened around a second shaft (e.g., the sleeve shaft 280).

In some examples, as shown in FIGS. 14 and 15, the second seal 304 can be disposed within the intermediate portion 348 of the second seal housing 308. The intermediate portion 348 can have a sixth inner diameter 362 which is larger than the fifth inner diameter 354.

In some examples, the second seal housing 308 can also include a transition portion 364 that includes a taper or angled step 366 that more gradually increases in diameter from the fifth inner diameter 354 to the sixth inner diameter 362. Further, in some instances, the angled step 366 can be annular and extend around the circumference of the second seal housing 308. In alternate examples, instead of being angled, the step of the transition portion 364 can be a right-angled step.

In some instances, the second seal 304 is shaped such that its proximal end portion tapers to match the taper or angling of the angled step 366. As such, the second seal 304 can be shaped to fit within the intermediate portion 348 and the transition portion 364, against the angled step 366.

It should be noted that although the step 358 is shown as extending to the transition portion 364, in alternate examples the step 358 can be shorter (in the axial direction) and be formed as a protrusion within the proximal portion 346. The angled step 366 can then taper from the larger sixth inner diameter 362 to a diameter that is larger than the fifth inner diameter 354.

The distal portion 350 of the second seal housing 308 has a seventh inner diameter 368 and includes a plurality of internal threads 370 in an inner surface 372 of the second seal housing 308 (FIG. 15). As shown in FIGS. 14 and 15, the threads 370 are disposed distal to the second seal 304 in the second seal housing 308.

The sealing mechanism can further include a second threaded member 374 coupled to the distal portion 350 of the second seal housing 308 (FIGS. 14-16). In particular, the second threaded member 374 can include external threads 376 that are configured to mate with the internal threads 370 of the second seal housing 308. A second knob 378 (or alternative rotatable element) can be fixed to the second threaded member 374 and configured to rotate

(FIGS. 11-16). In some instances, the second knob 378 can be coupled or fixed to a distal end of the second threaded member 374 and disposed around the distal portion 350 of the second seal housing 308. Rotation of the second knob 378 can cause the second threaded member 374 to rotate relative to the second seal housing 308, thereby causing the second threaded member 374 to travel in an axial direction (relative to the central longitudinal axis 301). As the second threaded member 374 travels proximally (toward the first seal housing 306), a proximal end 380 of the second threaded member 374 can contact and push against a distal end 382 of the second seal 304 (FIGS. 14 and 15), thereby compressing the second seal 304 around a shaft disposed therein (e.g., the sleeve shaft 280 shown in FIG. 14). In this way, the second seal 304 can be tightened around and sealed against a shaft disposed therein by rotating the second knob 378 (and consequently the second threaded member 374). Additionally, compressing the second seal 304 with the second knob 378 can also axially lock the sealing mechanism 300 to the shaft disposed therein, thereby ensuring the sealing mechanism 300 stays connected to the shaft during flushing at relatively high fluid pressures, as described below.

Inner surfaces of the first threaded member 336 and the first seal 302 can define a first lumen 384 of the sealing mechanism 300 having a first diameter 385 that is configured (shaped) to receive a first shaft (e.g., the outer shaft 260 shown in FIG. 14). Inner surfaces of the second threaded member 374 and the second seal 304 can define a second lumen 386 of the sealing mechanism 300 having a second diameter 387 that is configured (shaped) to receive a second shaft (e.g., the sleeve shaft 280 shown in FIG. 14). The second diameter 387 can be smaller than the first diameter 385.

FIG. 17 is a flow chart of an exemplary method 400 for selectively directing fluid flow through a catheter comprising multiple shafts that are at least partially concentric with one another (or one arranged within the other with slightly offset central longitudinal axes). In particular, the method 400 is a method for operating the sealing mechanism 300 of FIGS. 11-16 to block fluid flow out of a first shaft of the catheter and directing the fluid flow through a second shaft of the catheter. However, the method 400 can also be a method for operating the other sealing mechanisms described herein, such as the sealing mechanism 500 or the sealing mechanism 600. In some examples, the catheter can be the delivery apparatus 200 of FIGS. 6-10, the first shaft can be the outer shaft 260, and the second shaft can be the sleeve shaft 280.

Method 400 begins at 402 and includes attaching the first seal 302 of the sealing mechanism 300 to a distal end portion of a first shaft of a catheter (e.g., outer shaft 260, as shown in FIG. 14). Attaching the first seal 302 to the first shaft can include extending the distal end portion of the first shaft into the first lumen 384 of the sealing mechanism 300, through the first seal 302, and into the cavity 360 of the sealing mechanism 300 (e.g., as shown in FIG. 14). Additionally, in some examples, the distal end of the first shaft can hit or contact a stop in the second seal housing 308 (e.g., the step 358). Attaching the first seal 302 to the shaft can further include tightening the first seal 302 around the first shaft, for example, by rotating the first knob 340 and the first threaded member 336.

At 404, the method includes attaching the second seal 304 of a sealing mechanism 300 to a distal end portion of a second shaft of the catheter that extends through the first shaft (e.g., the sleeve shaft 280, as shown in FIG. 14), where the distal end portion of the second shaft extends distal to the distal end of the first shaft. For example, attaching the second seal 304 to the second shaft can include extending the distal end portion of the second shaft through and distal to the distal end of the first shaft and through the second seal. In some examples, the distal end of the second shaft can extend out a distal end of the sealing mechanism 300. Attaching the second seal 304 to the second shaft can further include tightening the second seal 304 around the second shaft, for example, by rotating the second knob 378 and the second threaded member 374. In alternate examples, when the second seal is instead a non-actively compressible seal (such as an O-ring as in the sealing mechanism 500 or sealing mechanism 600), the method at 404 can include extending the distal end portion of the second shaft through the second seal, where the second seal snuggly fits around and seals against the second shaft.

After tightening the first seal 302 and the second seal 304, the cavity 360 can be fluidly sealed (e.g., no fluid can exit the cavity 360), thereby occluding the distal end of the first shaft such that no fluid from a first lumen of the first shaft can exit the cavity 360.

Method 400 continues to 406 which includes flowing fluid through the catheter such that the fluid flows out of only a second lumen of the second shaft and is blocked from flowing out of the first lumen of the first shaft (the first lumen defined between an outer surface of the second shaft and an inner surface of the first shaft). As a result, the second lumen of the second shaft can be flushed and de-aired completely. For example, when the second shaft is the sleeve shaft 280, flowing fluid through the catheter and through only the second lumen (and not the first lumen), the docking device disposed within the sleeve shaft can be effectively and efficiently de-aired prior to an implantation procedure.

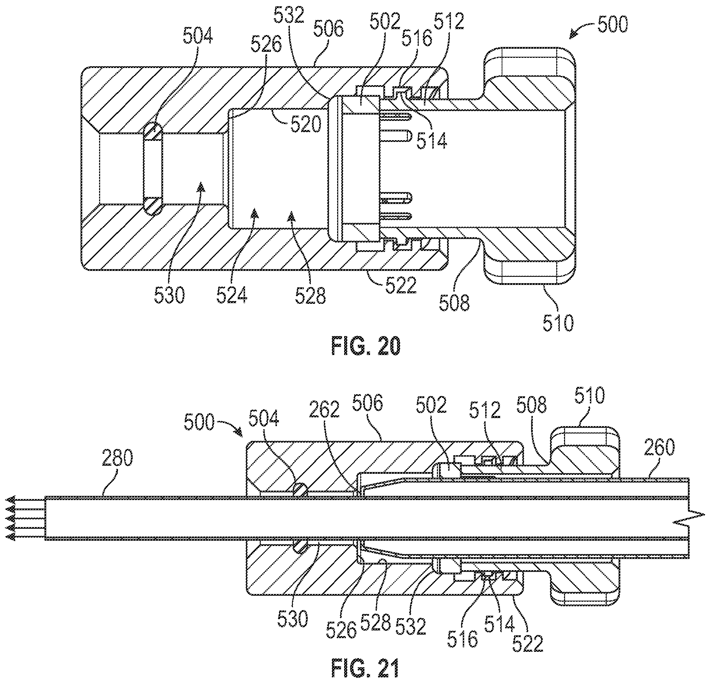

FIGS. 18-21 show an exemplary sealing mechanism 500 for a catheter that is configured to regulate fluid flow through two shafts of the catheter. FIGS. 18 and 19 are perspective views of the sealing mechanism 500. FIG. 20 is a cross-sectional side view of the sealing mechanism 500. FIG. 21 is another cross-sectional side view showing the sealing mechanism 500 coupled to the outer shaft 260 and the sleeve shaft 280 of the delivery apparatus 200. However, in alternate examples, the sealing mechanism 500 can be used with a variety of catheters and delivery apparatuses including two or more shafts with fluidly coupled lumens.

The sealing mechanism 500 can be similar to the sealing mechanism 300, except instead of two compressible seals (or gaskets) that are compressible around respective shafts via rotatable elements, the sealing mechanism 500 can comprise a compressible seal or gasket that is compressible around a first shaft of the catheter and a non-actively compressible seal (such as an O-ring) disposed around a second shaft of the catheter.

Referring to FIGS. 20-21, the sealing mechanism 500 includes a first seal 502 and a second seal 504 disposed within a housing 506 of the sealing mechanism 300. The first seal 502 and the second seal 504 can be annular with an aperture (for example, a central aperture) configured to receive a shaft therethrough.

In some examples, the first seal 502 is a compressible seal or gasket that is configured to be compressed around an outer shaft (such as the outer shaft 260) via a rotatable element 508 in a similar fashion to the first knob 340 and first threaded member 336 of sealing mechanism 300. In some examples, the second seal 504 is a non-actively compressible seal, such as an O-ring, that is shaped to snuggly fit around and seal against an inner shaft (such as the sleeve shaft 280). As used herein, the term non-actively means "without additional interaction (e.g., rotating, clamping, etc.) provided by a user and/or other mechanism."

The rotatable element 508 can also be configured to axially lock the sealing mechanism 500 in place as the system if pressurized. The compressible seal creating the axial lock can be disposed on the outer shaft 260 instead of the sleeve shaft 280 because in at least some instances the sleeve shaft 280 can have a hydrophilic coating that may result in reduced axial retention of the seal and rotatable element.

The rotatable element 508 can comprise a rotatable knob 510 and a threaded member 512 extending distally from the rotatable knob 510. The threaded member 512 can include one or more external threads 514 (or protrusions) (FIG. 19) that are configured to interface with internal threads 516 on an inner surface 520 of the housing (FIGS. 19-21). **In** some examples, as shown in FIG. 19, the external threads 514 can be discontinuous protrusions, spaced apart from one another around an outer surface of the threaded member 512, that are shaped to interface with and slide along the internal threads 516. **In** some examples, the threaded member 512 can also include one or more locking elements 518 (for example, tabs or cantilevered protrusions) that are configured to snap into engagement with the internal threads 516 and maintain the rotatable element 508 connected to the housing 506 (for example, without disengaging from the housing 506 when the rotatable element 508 is fully loosened).

The rotatable element 508 can be rotatable relative to the housing 506 such that the threaded member 512 moves distally against the first seal 502, pushes the first seal 502 axially against a curved edge 532 (or ramped edge) of the housing 506, which in turn compresses the first seal 502 radially against a shaft disposed therein, thereby tightening the first seal 502 around the shaft (for example, the outer shaft 260 as shown in FIG. 21). It should be noted that the radial compression of the first seal 502 can be more pronounced than that shown in FIG. 21, and in some examples, the first seal 502 can press further against the curved edge 532 and have a smaller inner diameter when being pushed axially against the housing 506.

The internal threads 516 of the housing 506 can be disposed at a first end portion 522 of the housing 506 which is proximal to a cavity 524 defined by the inner surface 520 of the housing 506 (FIG. 20). The first seal 502 can be disposed within the housing 506, adjacent and distal to the internal threads 516.

The inner surface 520 of the housing 506 can further define a step 526 within the cavity 524 that decreases a diameter of the cavity 524 from a larger diameter portion 528 of the cavity 524 to a smaller diameter portion 530 of the cavity 524 (FIGS. 20 and 21). The first seal 502 is disposed within the housing 506 adjacent and proximal to the larger diameter portion 528 of the cavity 524 and the second seal 504 is disposed within the housing 506 adjacent and distal to the smaller diameter portion 530 of the cavity 524 (FIGS. 20 and 21). In this way, the cavity 524 can be defined between the first seal 502 and the second seal 504.

Similar to as described above for the sealing mechanism 300, the step 526 can serve as a stop for the distal end 262 of the outer shaft 260 (FIG. 21). As such, the distal end 262 of the outer shaft 260 can be received within the larger diameter portion 528 of the cavity 524 and abut the step 526 with the first seal 502 sealing around the distal end portion of the outer shaft 260 (for example, as the rotatable element 508 is rotated, it pushes the first seal 502 axially against the housing (the curved edge 532, for example), which in turn compresses the first seal 502 against the outer shaft 260). The inner shaft or sleeve shaft 280 can then extend through and distal to the distal end 262 of the outer shaft 260, and through the smaller diameter portion 530 of the cavity 524 and the second seal 504. The second seal 504 can be sized to tightly fit around the outer surface of the sleeve shaft 280 such that it fluidly seals around the sleeve shaft 280. Thus, fluid passing through the delivery apparatus is blocked from exiting the distal end of the outer shaft 260 and instead forced through the sleeve shaft 280, as shown in FIG. 21 and described above with reference to the sealing mechanism 300 and the method 400.

FIGS. 22 and 23 show an exemplary sealing mechanism 600 for a catheter that is configured to regulate fluid flow through two shafts of the catheter. The sealing mechanism 600 can be similar to the sealing mechanism 600, except instead of one compressible seal or gasket that is compressible around an outer shaft and one non-compressible seal (such as an O-ring) that is configured to seal around an inner shaft, the sealing mechanism 600 can comprise two non-actively compressible seals (such as two O-rings).

For example, as shown in FIGS. 22 and 23, the sealing mechanism 600 can comprise a housing 602 (FIG. 22), a first seal 604 disposed within a first end portion 608 of the housing 602, and a second seal 606 disposed within a second end portion 610 of the housing 602 (FIG. 23). The first seal 604 can be larger than the second seal 606. For example, a first inner diameter 605 of the first seal 604 can be larger than a second inner diameter 607 of the second seal 606, where the first inner diameter 605 is shaped to receive and seal around a first shaft (such as the outer shaft 260) and the second inner diameter 607 is shaped to receive and seal around a second shaft (such as the sleeve shaft 280).

Similar to the sealing mechanisms described above, an inner surface 612 of the housing 602 defines a cavity 614 that is disposed between the first seal 604 and the second seal 606 (FIG. 23). The housing 602 can further define a step 616 within the cavity 614 that decreases a diameter of the cavity 614 from a larger diameter portion 620 to a smaller diameter portion 618 of the cavity 614.

The first seal 604 can be configured to snuggly fit around and seal against an outer surface of an outer shaft of the catheter (for example, the outer shaft 260), and the step 616 can form a stop for a distal end of the outer shaft. The second seal 606 can be configured to snuggly fit around and seal against an inner shaft of the catheter (for example, the sleeve shaft 280). In this way, the first seal 604 and second seal 606 can fluidly seal against the respective shafts of the catheter, without utilizing a rotatable element or knob, and a distal end of the outer shaft of the catheter can reside in the cavity 614 that is disposed between the first seal 604 and the second seal 606. As a result, flow out of the outer shaft is blocked, thereby forcing all or a majority of the flushing fluid introduced into the outer shaft to exist through the lumen of the inner shaft (for example, the sleeve shaft lumen of the sleeve shaft 280).

In some examples, it may be desirable to aspirate fluid from the distal end of the inner shaft of the catheter (for example, the sleeve shaft 280), rather than flushing through the catheter and sealing mechanism, as described above. Aspiration of fluid can be referred to herein as applying a negative pressure, at an end of a shaft for example, such that a vacuum is created, and fluid is pulled (rather than pushed) out of the shaft. In contrast, flushing fluid, as used herein, can refer to using positive fluid pressure to push fluid through a shaft. In some examples, the fluid aspiration and flushing techniques described herein may be used together to direct fluid through one or more shafts of a catheter.

In such fluid aspiration examples, a housing of any of the sealing mechanisms described above can extend distally from the second seal and include a second cavity and second step, both disposed distal to the second seal. For example, FIG. 24 shows the sealing mechanism 500 with the housing 506 further comprising a second cavity 550 disposed distal to the second seal 504 and a step 552 disposed within the second cavity 550. The step 552 decreases a diameter of the second cavity 550 from a larger first diameter adjacent to the second seal 504 to a smaller second diameter. The step 552 can serve as a stop for a distal end of the inner shaft (sleeve shaft 280).

A luer attachment 554 can be attached to the housing 506 distal to the second cavity 550. The luer attachment 554 can be configured to receive an aspiration tool 556 (such as a syringe) for creating a vacuum within the second cavity 550 and aspirating the inner shaft. In some examples, an extension tube 558 can be connected between the luer attachment 554 and the aspiration tool 556.

In some examples, a method for aspirating the inner shaft of the catheter can include, with the inner shaft (sleeve shaft 280) fully retracted inside the outer shaft (outer shaft 260), the sealing mechanism (for example, sealing mechanism 500 shown in FIG. 24) can be positioned around the outer shaft, such that the distal end of the outer shaft abuts the step 526. If using the sealing mechanism configuration with the rotatable element, the rotatable element 508 can be rotated such that the first seal 502 is axially compressed against the housing, thereby radially compressing the first seal 502 and tightening the first seal 502 around the outer shaft. The inner shaft (sleeve shaft 280) can then be advanced through the second seal 504 and into the second cavity 550, against the step 552. Finally, the aspiration tool 556 (for example, a syringe) can be attached to the luer attachment 554 and the user can pull a vacuum with the aspiration tool 556 to aspirate the catheter.

FIGS. 25 and 26 show an example of a sealing mechanism 700 for sealing to a shaft of a catheter and aspirating fluid out of the shaft. The sealing mechanism 700 can comprise a clamshell member 702 that is configured to open and receive the shaft of the catheter therein (for example, the sleeve shaft 280), as shown in FIG. 25. For example, the clamshell member 702 can comprise a first half shell 704 and a second half shel1 706 comprising a slot or lumen 720 for receiving the shaft. In some examples, the second half shell 706 can pivot relative to the first half shell 704 via a pivot joint 710 which is connected to a housing 712 of the sealing mechanism 700. As such, the second half shell 706 can pivot away from the first half shell 704 (into an open configuration, as shown in FIG. 25) in order to receive the shaft therein and then pivot toward and against the first half shell 704 (into a closed configuration, as shown in FIG. 25) to seal the shaft between the first half shell 704 and the second half shell 706.

In some examples, the first half shell 704 and the second half shell 706 can comprise a compressible padding 708 (such as a silicone pad or another compressible polymeric padding) that is configured to seal around the shaft when the clamshell member 702 is closed and clamped down around the shaft. The lumen 720 can be defined in the first half shell 704 and second half shell 706 that is configured to receive the catheter shaft therein.

A tube 714 can extend from the housing 712 and include a luer attachment 716 (or another type of mechanical attachment) that is configured to receive an aspiration tool (such as a syringe). A lumen of the tube 714 can be fluidly connected to the lumen 720, through a lumen of the housing 712.

In some examples, the sealing mechanism 700 can comprise a locking mechanism, configured as a sliding knob 718 that is axially slidable from a first position around a portion of an outer surface of the housing 712 (FIG. 25) to a second position around the closed first half shell 704 and second half shell 706 (FIG. 26). In the second position, the sliding knob 718 circumscribes the first half shell 704 and second half shell 706, thereby locking them in the closed and sealed position around the shaft. In some examples, the first half shell 704 and/or the second half shell 706 can include a stopping element 722 that is configured to stop the sliding knob 718 from traveling further toward the end of the first half shell 704 and the second half shell 706 (FIG. 26). Additionally or alternatively, in some examples, the sliding knob 718 can have an ergonomic grip around its outer surface (as shown in FIGS. 25 and 26).

In alternate examples, the sealing mechanism 700 can include an additional or alternative locking mechanism. For example, in alternative examples, instead of sliding, the knob 718 could be rotatable and have internal threads that interface with threads on the first half shell 704 and the second half shell 706. Thus, the rotatable knob could rotate and be threaded over the first half shell 704 and the second half shell 706 to hold them together in the closed and sealed position.

In alternate examples, in lieu of or in addition to the sliding knob 718, the first half shell 704 and the second half shell 706 could have complementary locking elements, such as ramped tabs, that allow the first half shell 704 and second half shell 706 to snap together (and be held together in the closed position until being released by a release mechanism, such as tabs that are pressed together for release).

In some examples, the first half shell 704 and the second half shell 706 can be spring loaded by a spring (e.g., a torsional spring). For example, in some instances, the first half shell 704 and the second half shell 706 can be spring loaded by a spring such that they are forced open by the spring and then can be closed together under pressure and kept closed by a locking mechanism (e.g., the sliding knob 718).

In some examples, the first half shell 704 and the second half shell 706 can be spring loaded by a spring such that they are forced closed by the spring and then can be opened and moved apart manually by a user (therefore negating a need for a locking mechanism in this instance).

Once the shaft (for example, the sleeve shaft 280) is closed and sealed within the clamshell member 702, as shown in FIG. 26, an aspiration tool can be connected to the tube 714 and used to pull a vacuum and aspirate fluid through and out of the shaft.

In this way, the sealing mechanism 700 can easily connect and seal to the shaft in need of flushing (the sleeve shaft 280). In some examples, the shaft can extend out of and distal to an outer shaft of the catheter (for example, the outer shaft 260) during the aspiration process.

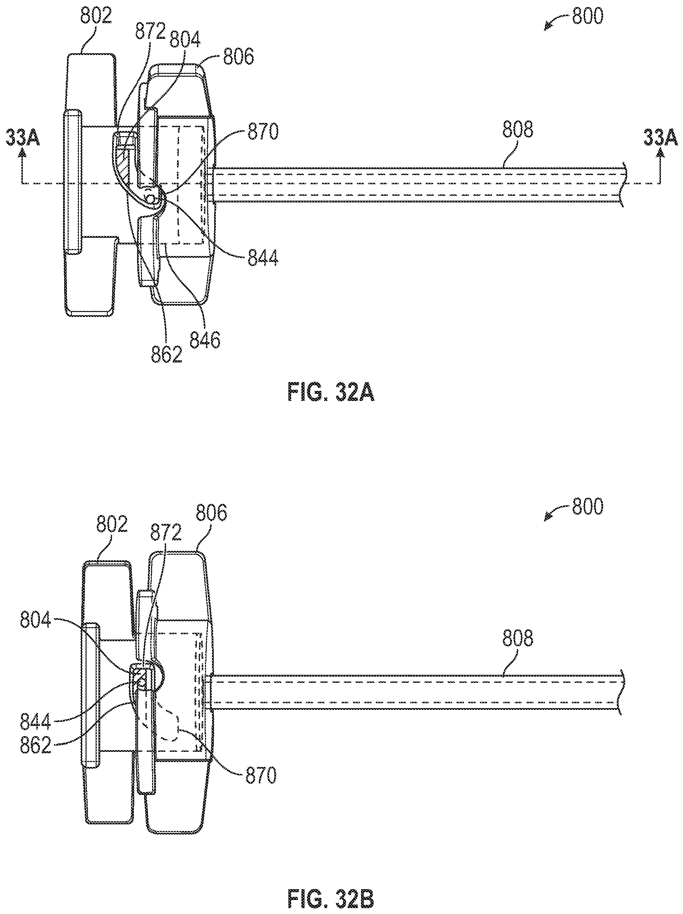

FIGS. 27-34 show an example of a sealing mechanism 800 (or sealing assembly) for sealing to a shaft of a catheter and aspirating fluid out of the shaft and/or flushing fluid through the shaft. The sealing mechanism 800 comprises a seal housing 802, a seal 804 disposed within the seal housing 802, and a lock cap 806 (also referred to herein as a locking cap, lock member, or locking member) configured to interface with the seal housing 802 and the seal 804. The lock cap 806 is rotatable relative to the seal housing 802 (or vice versa) about a central longitudinal axis 805 to move the sealing mechanism between an unlocked configuration (see, e.g., FIG. 32A) and a locked configuration (see, e.g., FIG. 32B).

In some examples, the sealing mechanism 800 can further comprise a tube 808 that extends distally from the lock cap 806. In some instances, the tube 808 is a flexible tube comprising a flexible or compliant material configured to receive the shaft of the catheter therethrough (and allow movement/bending of the shaft therein). For example, the tube 808 can be configured to take on a shape (e.g., a curved shape and/or a serpentine shape) of the inserted catheter shaft.

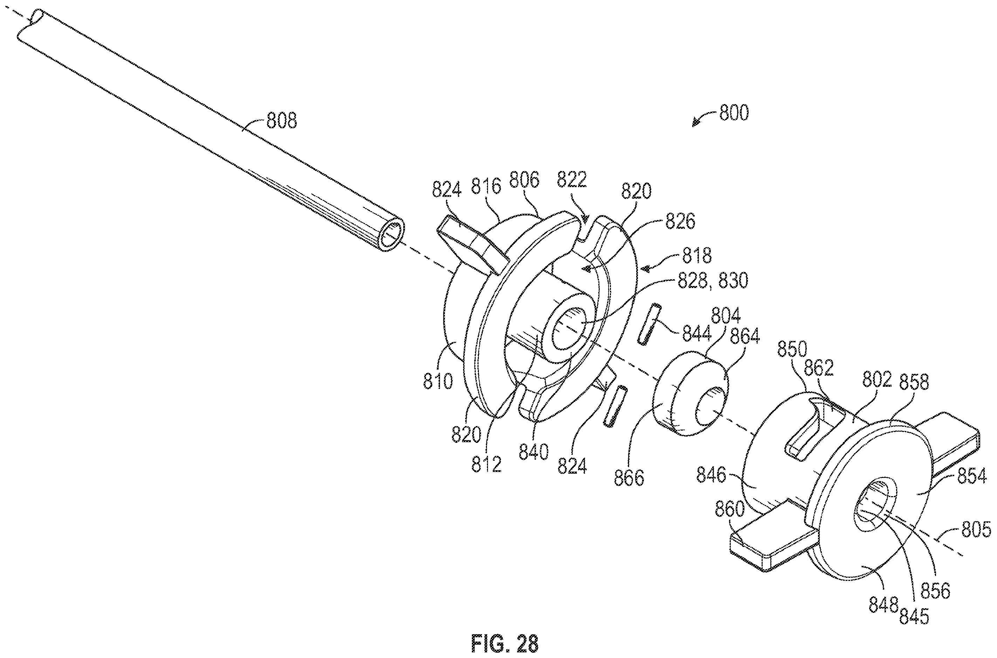

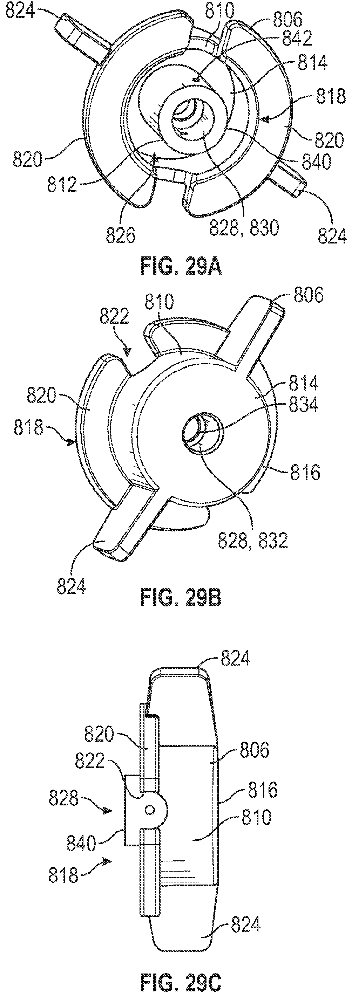

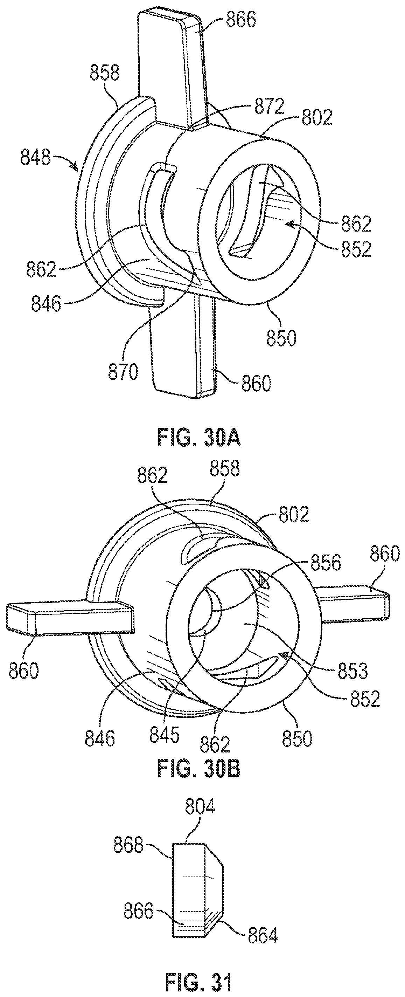

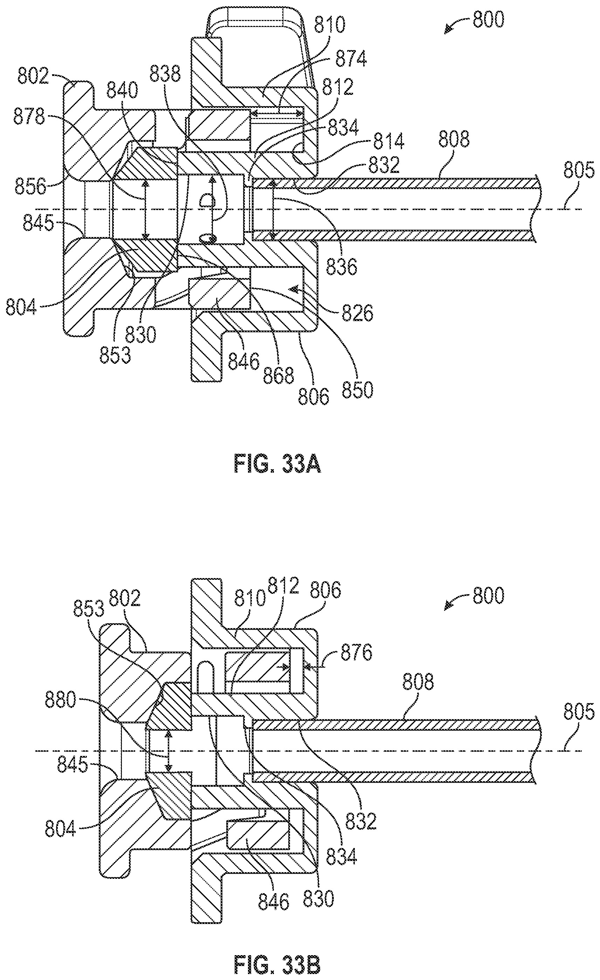

FIG. 27 shows an assembled view of the sealing mechanism 800, and FIG. 28 shows an exploded view of the sealing mechanism 800. The central longitudinal axis 805 of the sealing mechanism 800 is shown in FIGS. 27 and 28. The lock cap 806 is shown alone in FIGS. 29A-29C, the seal housing 802 is shown alone in FIGS. 30A and 30B, and the seal 804 is shown alone in FIG. 31. Additionally, FIGS. 32A and 32B depict side views of the sealing mechanism 800 and FIGS. 33A and 33B depict cross-sectional views of the sealing mechanism 800.

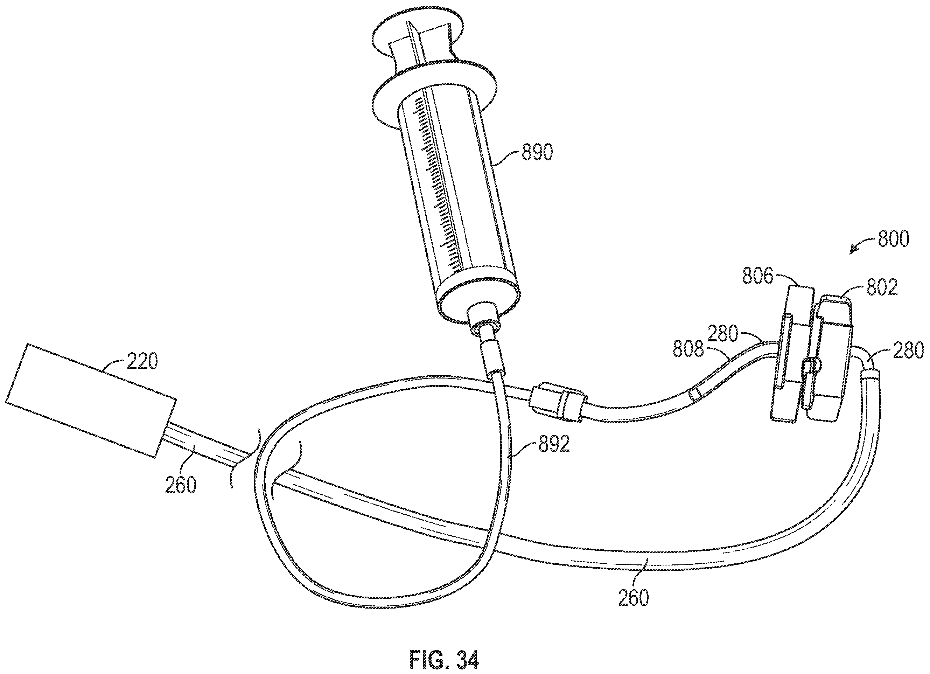

In some examples, the sealing mechanism 800 can seal a shaft of a delivery apparatus for an implantable medical device, such as the delivery apparatus 200 of FIGS. 6-10. For example, FIG. 34 shows the sealing mechanism 800 coupled to and sealed around the sleeve shaft 280 of the delivery apparatus 200. The sealing mechanism 800 can also be used with (and/or adapted for use with) a variety of catheters and delivery apparatuses including two or more shafts with fluidly coupled lumens.

The lock cap 806 is rotatable relative to the seal housing 802 (or the lock cap 806 and the seal housing 802 are rotatable relative to one another or the seal housing 802 is rotatable relative to the lock cap 806) such that the sealing mechanism 800 is moveable between an unlocked configuration (FIGS. 32A and 33A) and a locked configuration (FIGS. 32B, 33B, and 34) where the seal 804 is compressed (and held) tightly around a shaft arranged within and extending through the sealing mechanism 800. In this way, the sealing mechanism 800 can be used to flush or pull fluid through the shaft, thereby de-airing the shaft (and/or components arranged within the shaft).

As shown in FIGS. 27-29C, the lock cap 806 includes an outer wall 810 (or outer portion) and an inner wall 812 (or inner portion) (FIGS. 28 and 29A) extending proximally from an end wall 814 that defines a distal end 816 (or second end) of the lock cap 806. The outer wall 810 and the inner wall 812 can extend to an open, proximal end 818 (or first end) of the lock cap 806. A cross-section of the outer wall 810 and the inner wall 812 is annular. Thus, the outer wall 810 and the inner wall 812 can be referred to herein as circumferentially extending walls and/or annular walls.

In some instances, a proximal end of the outer wall 810, at the proximal end 818, can include one or more flanges 820 extending radially outward from the outer wall 810 and circumferentially around at least a portion of the outer wall 810 and lock cap 806. For example, as shown in FIGS. 27-29C, the lock cap 806 can include two circumferentially extending flanges 820 that are separated from one another in the circumferential direction by gaps 822 (or spaces). In this way, each flange 820 can extend around at least a portion of a circumference of the outer wall 810 (such as at least or greater than 1/3 of the total circumference).

In some instances, the lock cap 806 can comprise more or less than two flanges 820 (such as one, three, or the like). In some instances, a width of the gaps 822 (in the circumferential direction) can be larger or smaller than shown in FIGS. 27-29C.

In some instances, the lock cap 806 comprises one or more extensions 824 (or wings) that extend radially outwardly from the outer wall 810. The one or more extensions 824 are configured to be gripped by a user for rotating the sealing mechanism 800 into the locked and unlocked configurations. Each extension 824 can intersect one of the flanges 820. In some instances, as shown in FIGS. 27-29C, the extensions 824 extend further radially outwardly, relative to the central longitudinal axis 805, than the flanges 820.

For example, as shown in FIGS. 27-29C, the lock cap 806 can include two extensions 824 separated from one another (circumferentially) and disposed on opposite sides of the lock cap 806 (e.g., across the central longitudinal axis 805 from one another). Thus, the extensions 824 can extend radially outward from the outer wall 810, relative to the central longitudinal axis 805, in opposite directions.

In some instances, the lock cap 806 can comprise more or less than two extensions 824 (such as one, three, or the like).

A cavity 826 is defined between, in the radial direction (relative to the central longitudinal axis 805), the outer wall 810 and the inner wall 812 (FIGS. 28 and 29A). Thus, the cavity 826 can be formed by a space separating an outer surface of the inner wall 812 and an inner surface of the outer wall 810. As described further below, the cavity 826 can be configured to receive a portion of the seal housing 802 therein.

A lumen 828 is defined by an inner surface of the inner wall 812. The lumen 828 extends through the lock cap 806 and is configured to receive the shaft of the catheter (to be sealed against) therethrough. For example, the lumen 828 can comprise a first lumen portion 830 extending distally from the proximal end 818 that is configured to receive the shaft therethrough (FIGS. 28 and 29A).

In some instances, the lumen 828 also comprises a second lumen portion 832 extending proximally from the distal end 816 that is configured to receive the tube 808 therein. The tube 808 is configured to receive the shaft of the catheter therethrough. In this way, tube 808 can extend into the second lumen portion 832 and be coupled to the inner wall 812 of the lock cap 806. As such, when arranged within the second lumen portion 832, the tube 808 can extend distally outward from the distal end 606 of the lock cap 806 (as shown in FIG. 27).

In some instances, the inner surface of the inner wall 812 can define a step or annular protrusion 834 that separates the first lumen portion 830 and the second lumen portion 832 (FIG. 29B, and also shown in FIGS. 33A and 33B). In some instances, the second lumen portion 832 has a second diameter 836 that is smaller than a first diameter 838 of the first lumen portion 830 (as shown in FIG. 33A).

The inner wall 812 has an axially facing proximal surface 840 at the proximal end 818 which is configured to interface with the seal 804 (as described further below).

In some examples, the inner wall 812 also includes one or more radially extending channels 842 (or apertures) that extend between the inner surface and outer surface of the inner wall 812 (FIG. 29A). The one or more channels 842 (e.g., two are shown in FIG. 29A) are configured to receive one or more pins 844 (FIG. 28) which form a locking assembly of the sealing mechanism 800 with the seal housing 802 (as described further below). In this way, the pins 844 can extend through the respective channels 842 and couple to the lock cap 806.

In some examples, the pins 844 can be an integral part of the lock cap 806. For example, the pins 844 can be mounted within and affixed to corresponding channels 842 in the lock cap 806. In some examples, instead of being disposed within and protruding outwardly from the channels 842, the pins 844 can be affixed to and protrude radially outward from an outer surface of the inner wall 812. In this way, the pins 844 can be extensions of the inner wall 812 in some examples.

Turning to FIGS. 30A and 30B (and also FIGS. 27 and 28), the seal housing 802 comprises a cylindrical body portion 846 extending between a proximal end 848 (FIGS. 27 and 28) and a distal end 850 (FIGS. 30A and 30B) of the seal housing 802. An inner surface of the cylindrical body portion 846 defines a cavity 852 therein. An axially facing, proximal wall 854 (FIGS. 27 and 28) is formed at the proximal end 848 of the cylindrical body portion 846 and defines an opening 856 that is configured (e.g., shaped and/or sized) to receive the catheter shaft therethrough. A diameter of the opening 856 is smaller than a diameter of the cylindrical body portion 846 at the distal end 850.

For example, the inner surface of the cylindrical body portion 846 can further define a lumen 845 extending into the cylindrical body portion 846 from the opening 856. The lumen 845 opens up into the wider (larger diameter) cavity 852 at a ramped surface 853 (FIGS. 30B, 33A, and 33B) defined by the inner surface of the cylindrical body portion 846. The ramped surface 853 can be shaped to receive the seal 804 therein, as described further below with reference to FIGS. 33A and 33B. The ramped surface 853 is angled at a non-zero angle relative to the central longitudinal axis 805.

In some instances, as shown in FIGS. 27-28 and 30A-30B, the proximal wall 854 has a circumferentially extending extension or flange 858 that extends radially outward from an outer surface of the cylindrical body portion 846 and extends around at least a portion of the circumference of the cylindrical body portion 846.

In some instances, the seal housing 802 includes one or more radially extending extensions 860 (or flanges) that extend radially outward from the cylindrical body portion 846. The one or more extensions 860 are configured to be gripped by a user for holding and/or rotating the seal housing 802 relative to the lock cap 806 when moving the sealing mechanism 800 between the locked and unlocked configurations. In some examples, the one or more extensions 860 can intersect the flange 858. The extensions 860 extend further radially outward, relative to the central longitudinal axis 805, than the flange 858.

Though two extensions 860 are depicted in FIGS. 27-28, 30A-30B, and 32A-34, in alternate examples, the seal housing 802 can comprise more or less than two extensions 860 (e.g., one, three, four, or the like).

The cylindrical body portion 846 includes one or more slots 862 (or at least one slot 862) extending therethrough, between an inner surface and an outer surface of the cylindrical body portion 846 (e.g., through a thickness of the cylindrical body portion 846, as defined in the radial direction). For example, as shown in FIGS. 27-28, and 30A-30B, the cylindrical body portion 846 includes two slots 862 spaced circumferentially apart from one another (for example, 180 degrees apart in some instances). Each slot 862 can be configured to receive one of the pins 844, as shown in FIG. 27 and FIGS. 32A and 32B (as described in greater detail below). Thus, the number of slots 862 is equal to the number of pins 844. In alternate examples, more or less than two slots 862 and pins 844 are possible, with the number of pins 844 and slots 862 being equal.

As shown in FIGS. 28, 30A, and 30B, each slot 862 can have a non-straight shape, such as curved. For example, each slot 862 can have a circumferentially extending portion at a second end 872 of the slot 862 and an axially extending portion at a first end 870 of the slot 862. The first end 870 and the second end 872 of the slot 862 are opposite ends of the slot 862. The first end 870 is closer to the distal end 850 of the cylindrical body portion 846 than the second end 872 of each slot 862 (FIG. 30A).

As such, when the seal housing 802 and lock cap 806 are rotated relative to one another between the locked and unlocked configurations, each pin 844 can slide within a respective slot 862 (between the opposing first and second ends 870, 872) and correspondingly cause axial movement of the seal housing 802 and lock cap 806 toward and away from one another, respectively (as described further below). Such relative movement between the seal housing 802 and lock cap 806 causes the seal 804 to be axially compressed between the seal housing 802 and lock cap 806 and radially compressed around (and/or expanded against) a shaft extending through the sealing mechanism 800.

The pins 844 and corresponding slots 862 can be configured such that the seal housing 802 and the lock cap 806 rotate relative to one another less than 360 degrees, 45-225 degrees, 70-200 degrees, 170-190 degrees, or 80-100 degrees between the unlocked configuration and the locked configuration.

A side view of the seal 804 is shown in FIG. 31. The seal 804 comprises a proximal portion 864 and a distal portion 866. The distal portion 866 is cylindrical (annular). In some examples, the proximal portion 864 is angled or ramped such that its outer diameter decreases from the distal portion 866 to a proximal end of the proximal portion 864. A diameter of a lumen of the seal 804 can be relatively constant through the seal 804 (through the distal portion 866 and the proximal portion 864). In this way, the seal 804 can be configured to fit within the cavity 852 of the seal housing 802 and the angled outer surface of the proximal portion 864 can be configured to interface with and fit against the ramped surface 853. An axially facing distal face 868 at the distal end of the distal portion 866 is configured to interface with (and have face-to-face contact with) the proximal surface 840 of the lock cap 806 (FIGS. 33A and 33B).

FIGS. 32A-33B depict operation of the sealing mechanism 800. As introduced above, a locking assembly of the sealing mechanism 800 is movable between an unlocked configuration (or position) (e.g., FIGS. 32A and 33A) and a locked configuration (or position) (e.g., FIGS. 32B and 33B). For example, the locking assembly can be formed by the pins 844 extending through (and/or coupled to) the channels 842 of the lock cap 806 and sliding along the slots 862. The seal housing 802 and lock cap 806 are rotated relative to one another to slide the pins 844 along the slots 862 between a fist end 870 of the slots 862 (as shown in FIG. 32A, in the unlocked configuration) and a second end 872 of the slots 862 (as shown in FIG. 32B, in the locked configuration). As such, the sealing mechanism 800 provides discrete, binary sealed/unsealed states, which can make the device easy to use.

In the unlocked configuration, the pins 844 are disposed at the first end 870 of the slots 862 (FIG. 32A) and an end wall 814 of the lock cap 806 and the distal end 850 of the seal housing 802 are spaced apart from one another by a first gap 874 (FIG. 33A). In this position, the distal face 868 of the seal 804 can abut or be disposed proximate to the proximal surface 840 of the inner wall 812 of the lock cap 806, but the seal 804 is an uncompressed state (for example, not compressed between the seal housing 802 and the inner wall 812 of the lock cap 806) or a less compressed state (such that it is not compressed against a catheter shaft extending therethrough). In this state, the ramped surface 853 of the cylindrical body portion 846 has a steeper angle and larger diameter than the corresponding portion of the seal 804 (and there is a gap between the ramped surface 853 and the seal 804, as shown in FIG. 33A). Thus, a shaft can be inserted into the sealing mechanism 800 in this configuration and the seal 804 is not compressed and sealed around the shaft.

As one example, to move the sealing mechanism 800 from the unlocked configuration (FIGS. 32A and 33A) to the locked configuration (FIGS. 32B and 33B), a user can hold the lock cap 806 stationary and rotate the seal housing 802 such that the pins 844 move along the slots 862 to the second ends 872 of the slots 862 and the seal housing 802 moves toward (in the axial direction) the lock cap 806.

In alternative examples, a user can rotate the seal housing 802 and lock cap 806 relative to one another (in opposite rotational directions), or the lock cap 806 relative to the seal housing 802, to move the sealing mechanism 800 into the locked configuration.

As the seal housing 802 moves closer to the lock cap 806, the seal 804 is pressed against the proximal surface 840 of the inner wall 812 of the lock cap 806 (and thereby compressed in the axial direction) and the seal 804 is forced radially outward to fill the space between the ramped surface 853 and the seal 804 and also radially inward toward the central longitudinal axis 805. Thus, when a shaft is arranged inside the sealing mechanism 800 (e.g., through the lumen 845 and a lumen of the tube 808), the axially compressed and radially expanded seal 804 presses against an outer surface of the shaft, thereby sealing against the shaft (and creating a fluid-tight seal). As shown in FIG. 33B, in the locked configuration, the lock cap 806 and the seal housing 802 are spaced apart from one another by a second gap 876 (FIG. 33B) that is smaller than the first gap 874. Additionally, a second diameter 878 of a lumen of the seal 804 in the locked configuration is smaller than a first diameter 880 of the lumen of the seal 804 in the unlocked configuration.

In some examples, as shown in FIG. 34, the sealing mechanism 800 can be used with the delivery apparatus 200. For example, when the sealing mechanism 800 is in the unlocked configuration, the sleeve shaft 280 (which is extended distal to a distal end of the outer shaft 260) is inserted into the seal housing 802, through the seal housing 802, through the lock cap 806, and into the flexible tube 808 (FIG. 34). The lock cap 806 and the seal housing 802 are then rotated relative to one another to move the sealing mechanism 800 into the sealed and locked configuration (as depicted in FIG. 34). An aspiration tool 890 can be attached to a distal end of the flexible tube 808 (at an attachment of the tube 808 or an extension tube 892 extending between the flexible tube 808 and the aspiration tool 890). Suction (or a vacuum) is then created with the aspiration tool 890 (e.g., by pulling back on a plunger of a syringe) to pull fluid through the catheter and out of the sleeve shaft 280, thereby aspirating the sleeve shaft 280.

In some examples, instead of being used for aspiration (or suction), the aspiration tool 890 can be filled with fluid and then used to push (and flush) fluid through the sleeve shaft 280 (or another catheter shaft disposed within the sealing mechanism 800).

Alternatively (or additionally), the end of the flexible tube 808 can be open (unattached to an aspiration tool) and fluid from a fluid source at a proximal end of the catheter (e.g., flushing ports 210, 216, and/or 218 in the handle assembly 220 as shown in FIGS. 6, 9, and 10) can be pushed through the catheter and into and through the sleeve shaft 280.

As a result, the sleeve shaft, or a shaft of an alternative catheter that is inserted into the sealing mechanism 800, can be effectively flushed and/or aspirated prior to use of the catheter during a procedure.

### Delivery Techniques

For implanting a prosthetic valve within the native aortic valve via a transfemoral delivery approach, the prosthetic valve is mounted in a radially compressed state along the distal end portion of a delivery apparatus. The prosthetic valve and the distal end portion of the delivery apparatus are inserted into a femoral artery and are advanced into and through the descending aorta, around the aortic arch, and through the ascending aorta. The prosthetic valve is positioned within the native aortic valve and radially expanded (e.g., by inflating a balloon, actuating one or more actuators of the delivery apparatus, or deploying the prosthetic valve from a sheath to allow the prosthetic valve to self-expand). Alternatively, a prosthetic valve can be implanted within the native aortic valve in a transapical procedure, whereby the prosthetic valve (on the distal end portion of the delivery apparatus) is introduced into the left ventricle through a surgical opening in the chest and the apex of the heart and the prosthetic valve is positioned within the native aortic valve. Alternatively, in a transaortic procedure, a prosthetic valve (on the distal end portion of the delivery apparatus) is introduced into the aorta through a surgical incision in the ascending aorta, such as through a partial J-sternotomy or right parasternal mini-thoracotomy, and then advanced through the ascending aorta toward the native aortic valve.

For implanting a prosthetic valve within the native mitral valve via a transseptal delivery approach, the prosthetic valve is mounted in a radially compressed state along the distal end portion of a delivery apparatus. The prosthetic valve and the distal end portion of the delivery apparatus are inserted into a femoral vein and are advanced into and through the inferior vena cava, into the right atrium, across the atrial septum (through a puncture made in the atrial septum), into the left atrium, and toward the native mitral valve. Alternatively, a prosthetic valve can be implanted within the native mitral valve in a transapical procedure, whereby the prosthetic valve (on the distal end portion of the delivery apparatus) is introduced into the left ventricle through a surgical opening in the chest and the apex of the heart and the prosthetic valve is positioned within the native mitral valve.

For implanting a prosthetic valve within the native tricuspid valve, the prosthetic valve is mounted in a radially compressed state along the distal end portion of a delivery apparatus. The prosthetic valve and the distal end portion of the delivery apparatus are inserted into a femoral vein and are advanced into and through the inferior vena cava, and into the right atrium, and the prosthetic valve is positioned within the native tricuspid valve. A similar approach can be used for implanting the prosthetic valve within the native pulmonary valve or the pulmonary artery, except that the prosthetic valve is advanced through the native tricuspid valve into the right ventricle and toward the pulmonary valve/pulmonary artery.

Another delivery approach is a transatrial approach whereby a prosthetic valve (on the distal end portion of the delivery apparatus) is inserted through an incision in the chest and an incision made through an atrial wall (of the right or left atrium) for accessing any of the native heart valves. Atrial delivery can also be made intravascularly, such as from a pulmonary vein. Still another delivery approach is a transventricular approach whereby a prosthetic valve (on the distal end portion of the delivery apparatus) is inserted through an incision in the chest and an incision made through the wall of the right ventricle (typically at or near the base of the heart) for implanting the prosthetic valve within the native tricuspid valve, the native pulmonary valve, or the pulmonary artery.

In all delivery approaches, the delivery apparatus can be advanced over a guidewire previously inserted into a patient's vasculature. Moreover, the disclosed delivery approaches are not intended to be limited. Any of the prosthetic valves disclosed herein can be implanted using any of various delivery procedures and delivery devices known in the art.

Any of the systems, devices, apparatuses, etc. herein can be sterilized (for example, with heat/thermal, pressure, steam, radiation, and/or chemicals, etc.) to ensure they are safe for use with patients, and any of the methods herein can include sterilization of the associated system, device, apparatus, etc. as one of the steps of the method. Examples of heat/thermal sterilization include steam sterilization and autoclaving. Examples of radiation for use in sterilization include, without limitation, gamma radiation, ultra-violet radiation, and electron beam. Examples of chemicals for use in sterilization include, without limitation, ethylene oxide, hydrogen peroxide, peracetic acid, formaldehyde, and glutaraldehyde. Sterilization with hydrogen peroxide may be accomplished using hydrogen peroxide plasma, for example.

The features described herein with regard to any example can be combined with other features described in any one or more of the other examples, unless otherwise stated. For example, any one or more of the features of one delivery apparatus can be combined with any one or more features of another delivery apparatus.

In view of the many possible ways in which the principles of the disclosure may be applied, it should be recognized that the illustrated configurations depict examples of the disclosed technology and should not be taken as limiting the scope of the disclosure nor the claims. Rather, the scope of the claimed subject matter is defined by the following claims.

## Claims

1. An assembly comprising:
a catheter comprising:
a first shaft (260); and
a second shaft (280) extending through the first shaft (260), wherein a first lumen is defined between an inner surface of the first shaft (260) and an outer surface of the second shaft (280); and
a sealing mechanism (300) comprising:
a first seal (302) disposed around a distal end portion of the first shaft (260);
a second seal (304) disposed around a portion of the second shaft (280) that extends distal to the first shaft (260); and
a cavity (360) disposed within a housing of the sealing mechanism (300) between the first seal (302) and the second seal (304), wherein a distal end of the first shaft (260) is disposed within the cavity (360), and wherein the cavity (360) is fluidly sealed by the first seal (302) and the second seal (304) such that fluid from the first lumen cannot exit the cavity (360).

2. The assembly of claim 1, wherein a distal end of the first lumen is occluded by the cavity (360), wherein the second shaft (280) has a second lumen, and wherein a distal end of the second lumen is open and extends distal to the second seal (304).

3. The assembly of either claim 1 or claim 2, wherein the sealing mechanism (300) comprises a step (358) within the cavity (360) that decreases a diameter of the cavity (360) from a larger first diameter (326) adjacent to the first seal (302) to a smaller second diameter (354) adjacent to the second seal (304), and wherein the distal end of the first shaft (260) is disposed against the step (358);
preferably wherein the housing comprises a first seal housing (306) containing the first seal (302) therein and a second seal housing (308) containing the second seal (304) therein, and wherein the step (358) is formed on an inner surface of the second seal housing (308).

4. The assembly of any one of claims 1 to 3, wherein the first seal (302) is disposed in a first seal housing (306) of the sealing mechanism (300) and the second seal (304) is disposed in a second seal housing (308) of the sealing mechanism (300), the first and second seal housings (308) coupled to one another, and wherein the cavity (360) is defined by an inner surface of the first seal housing (306) and an inner surface of the second seal housing (308).

5. The assembly of claim 4, wherein the sealing mechanism (300) further comprises a first threaded member (336) that interfaces with threads on the inner surface of the first seal housing (306) and is configured to rotate relative to the first seal housing (306) and tighten the first seal (302) around the first shaft (260);
preferably wherein the sealing mechanism (300) comprises a rotatable first knob (340) coupled to the first threaded member (336) and configured to rotate the first threaded member (336) such that it moves distally against the first seal (302) and tightens the first seal (302) around the first shaft (260).

6. The assembly of claim 5, wherein the sealing mechanism (300) further comprises a second threaded member (374) that interfaces with threads on the inner surface of the second seal housing (308) and is configured to rotate relative to the second seal housing (308) and tighten the second seal (304) around the second shaft (280);
preferably wherein the sealing mechanism (300) comprises a rotatable second knob (378) coupled to the second threaded member (374) and configured to rotate the second threaded member (374) such that it moves proximally against the second seal (304) and tightens the second seal (304) around the second shaft (280).

7. The assembly of claim 6, wherein the first threaded member (336) has a larger diameter lumen than the second threaded member (374).

8. The assembly of any one of claims 1 or 2, wherein the first seal (302) and the second seal (304) are disposed in the housing, and wherein the cavity (360) is defined by an inner surface of the housing.

9. The assembly of claim 8, wherein the sealing mechanism (300) further comprises a threaded member (512) that interfaces with threads on the inner surface of the housing at an end of the housing that is adjacent to the first seal (302), and wherein the threaded member (512) is configured to rotate relative to the housing and tighten the first seal (302) around the first shaft (260);
preferably:
wherein the sealing mechanism (300) comprises a rotatable knob (510) disposed at an end of the threaded member (512), and wherein the rotatable knob (510) is configured to rotate the threaded member (512) such that it moves distally against the first seal (302) and tightens the first seal (302) around the first shaft (260); and/or
wherein the first seal (302) is a compressible gasket (502), and the second seal (304) is an O-ring (504).

10. The assembly of claim 8, wherein the first seal (302) is an O-ring (504), and the second seal (304) is an O-ring (504).

11. The assembly of any one of claims 1 to 10, wherein the catheter is a delivery apparatus (200) for a docking device (232), and wherein the second shaft (280) is configured to contain the docking device (232) in a delivery configuration within the distal end portion of the second shaft (280).

12. A method for flushing a catheter comprising:
attaching a first seal (302) of a sealing mechanism (300) to a distal end portion of a first shaft (260) of a catheter;
attaching a second seal (304) of a sealing mechanism (300) to a distal end portion of a second shaft (280) of the catheter that extends through the first shaft (260), wherein the distal end portion of the second shaft (280) extends distal to a distal end of the first shaft (260); and
flowing fluid through the catheter such that the fluid flows out of only a second lumen defined by the second shaft (280) and is blocked from flowing out of a first lumen defined between an outer surface of the second shaft (280) and an inner surface of the first shaft (260).

13. The method of claim 12, wherein attaching the first seal (302) to the first shaft (260) includes extending the distal end portion of the first shaft (260) into a lumen of the sealing mechanism (300), through the first seal (302), and into a cavity (360) of the sealing mechanism (300) that is defined by walls of a housing of the sealing mechanism (300), between the first seal (302) and the second seal (304); and/or
wherein attaching the second seal (304) to the second shaft (280) includes extending the distal end portion of the second shaft (280) through and distal to the distal end of the first shaft (260) and through the second seal (304); and/or
wherein the attaching the first seal (302) and attaching the second seal (304) includes tightening the first seal (302) around the first shaft (260) and tightening the second seal (304) around the second shaft (280) such that the distal end of the first shaft (260) is occluded.

14. The method of any one of claims 12 or 13, wherein flowing fluid through the catheter such that the fluid flows out of only the second lumen defined by the second shaft (280) and is blocked from flowing out of the first lumen includes flushing fluid through the catheter using positive pressure applied to the catheter.

15. The method of any one of claims 12 or 13, wherein flowing fluid through the catheter such that the fluid flows out of only the second lumen defined by the second shaft (280) and is blocked from flowing out of the first lumen includes aspirating fluid through the catheter using negative pressure applied to a distal end of the second shaft (280) by an aspiration tool.

## Patentansprüche

1. Anordnung, umfassend:
einen Katheter, umfassend:
einen ersten Schaft (260); und
einen zweiten Schaft (280), der sich durch den ersten Schaft (260) erstreckt, wobei ein erstes Lumen zwischen einer inneren Oberfläche des ersten Schafts (260) und einer äußeren Oberfläche des zweiten Schafts (280) definiert ist; und
einen Abdichtmechanismus (300), umfassend:
eine erste Dichtung (302), die um einen distalen Endabschnitt des ersten Schafts (260) angeordnet ist;
eine zweite Dichtung (304), die um einen Abschnitt des zweiten Schafts (280) angeordnet ist, der sich distal zu dem ersten Schaft (260) erstreckt; und
einen Hohlraum (360), der innerhalb eines Gehäuses des Abdichtmechanismus (300) zwischen der ersten Dichtung (302) und der zweiten Dichtung (304) angeordnet ist, wobei ein distales Ende des ersten Schafts (260) innerhalb des Hohlraums (360) angeordnet ist, und wobei der Hohlraum (360) durch die erste Dichtung (302) und die zweite Dichtung (304) derart fluiddicht abgedichtet ist, dass Fluid aus dem ersten Lumen nicht aus dem Hohlraum (360) austreten kann.

2. Anordnung gemäß Anspruch 1, wobei ein distales Ende des ersten Lumens durch den Hohlraum (360) verschlossen ist, wobei der zweite Schaft (280) ein zweites Lumen aufweist, und wobei ein distales Ende des zweiten Lumens offen ist und sich distal zur zweiten Dichtung (304) erstreckt.

3. Anordnung gemäß Anspruch 1 oder Anspruch 2, wobei der Abdichtmechanismus (300) eine Stufe (358) innerhalb des Hohlraums (360) umfasst, die einen Durchmesser des Hohlraums (360) von einem größeren ersten Durchmesser (326) benachbart zur ersten Dichtung (302) auf einen kleineren zweiten Durchmesser (354) benachbart zur zweiten Dichtung (304) verringert, und wobei das distale Ende des ersten Schafts (260) an der Stufe (358) anliegend angeordnet ist;
vorzugsweise wobei das Gehäuse ein erstes Dichtungsgehäuse (306), das die erste Dichtung (302) darin enthält, und ein zweites Dichtungsgehäuse (308), das die zweite Dichtung (304) darin enthält, umfasst und wobei die Stufe (358) an einer inneren Oberfläche des zweiten Dichtungsgehäuses (308) ausgebildet ist.

4. Anordnung gemäß einem der Ansprüche 1 bis 3, wobei die erste Dichtung (302) in einem ersten Dichtungsgehäuse (306) des Abdichtmechanismus (300) angeordnet ist und die zweite Dichtung (304) in einem zweiten Dichtungsgehäuse (308) des Abdichtmechanismus (300) angeordnet ist, wobei das erste und das zweite Dichtungsgehäuse (308) miteinander gekoppelt sind, und wobei der Hohlraum (360) durch eine innere Oberfläche des ersten Dichtungsgehäuses (306) und eine innere Oberfläche des zweiten Dichtungsgehäuses (308) definiert ist.

5. Anordnung gemäß Anspruch 4, wobei der Abdichtmechanismus (300) ferner ein erstes Gewindeelement (336) umfasst, das mit einem Gewinde auf der inneren Oberfläche des ersten Dichtungsgehäuses (306) ineinander greift und dazu ausgelegt ist, sich relativ zu dem ersten Dichtungsgehäuse (306) zu drehen und die erste Dichtung (302) um den ersten Schaft (260) festzuziehen;
vorzugsweise wobei der Abdichtmechanismus (300) einen drehbaren ersten Knopf (340) umfasst, der mit dem ersten Gewindeelement (336) gekoppelt ist und dazu ausgelegt ist, das erste Gewindeelement (336) derart zu drehen, dass es sich distal gegen die erste Dichtung (302) bewegt und die erste Dichtung (302) um den ersten Schaft (260) festzieht.

6. Anordnung gemäß Anspruch 5, wobei der Abdichtmechanismus (300) ferner ein zweites Gewindeelement (374) umfasst, das mit einem Gewinde auf der inneren Oberfäche des zweiten Dichtungsgehäuses (308) ineinander greift und dazu ausgelegt ist, sich relativ zu dem zweiten Dichtungsgehäuse (308) zu drehen und die zweite Dichtung (304) um den zweiten Schaft (280) festzuziehen;
vorzugsweise wobei der Abdichtmechanismus (300) einen drehbaren zweiten Knopf (378) umfasst, der mit dem zweiten Gewindeelement (374) gekoppelt ist und dazu ausgelegt ist, das zweite Gewindeelement (374) derart zu drehen, dass es sich proximal gegen die zweite Dichtung (304) bewegt und die zweite Dichtung (304) um den zweiten Schaft (280) festzieht.

7. Anordnung gemäß Anspruch 6, wobei das erste Gewindeelement (336) ein Lumen mit größerem Durchmesser aufweist als das zweite Gewindeelement (374).

8. Anordnung gemäß einem der Ansprüche 1 oder 2, wobei die erste Dichtung (302) und die zweite Dichtung (304) in dem Gehäuse angeordnet sind, und wobei der Hohlraum (360) durch eine innere Oberfläche des Gehäuses definiert ist.

9. Anordnung gemäß Anspruch 8, wobei der Abdichtmechanismus (300) ferner ein Gewindeelement (512) umfasst, das mit einem Gewinde auf der inneren Oberfäche des Gehäuses an einem Ende des Gehäuses, das an die erste Dichtung (302) angrenzt, ineinander greift, und wobei das Gewindeelement (512) dazu ausgelegt ist, sich relativ zu dem Gehäuse zu drehen und die erste Dichtung (302) um den ersten Schaft (260) festzuziehen;
vorzugsweise:
wobei der Abdichtmechanismus (300) einen drehbaren Knopf (510) umfasst, der an einem Ende des Gewindeelements (512) angeordnet ist, und wobei der drehbare Knopf (510) dazu ausgelegt ist, das Gewindeelement (512) derart zu drehen, dass es sich distal gegen die erste Dichtung (302) bewegt und die erste Dichtung (302) um den ersten Schaft (260) festzieht; und/oder
wobei die erste Dichtung (302) eine komprimierbare Dichtung (502) ist und die zweite Dichtung (304) ein O-Ring (504) ist.

10. Anordnung gemäß Anspruch 8, wobei die erste Dichtung (302) ein O-Ring (504) ist und die zweite Dichtung (304) ein O-Ring (504) ist.

11. Anordnung gemäß einem der Ansprüche 1 bis 10, wobei der Katheter eine Zufuhrvorrichtung (200) für eine Dockingvorrichtung (232) ist, und wobei der zweite Schaft (280) dazu ausgelegt ist, die Dockingvorrichtung (232) in einer Zufuhrkonfiguration innerhalb des distalen Endabschnitts des zweiten Schafts (280) aufzunehmen.

12. Verfahren zum Spülen eines Katheters, umfassend:
Anbringen einer ersten Dichtung (302) eines Abdichtmechanismus (300) an einem distalen Endabschnitt eines ersten Schafts (260) eines Katheters;
Anbringen einer zweiten Dichtung (304) eines Abdichtmechanismus (300) an einem distalen Endabschnitt eines zweiten Schafts (280) des Katheters, der sich durch den ersten Schaft (260) erstreckt, wobei sich der distale Endabschnitt des zweiten Schafts (280) distal zu einem distalen Ende des ersten Schafts (260) erstreckt; und
Fließen von Fluid durch den Katheter derart, dass das Fluid nur aus einem zweiten Lumen, das durch den zweiten Schaft (280) definiert ist, ausströmt und daran gehindert wird, aus einem ersten Lumen auszuströmen, das zwischen einer äußeren Oberfläche des zweiten Schafts (280) und einer inneren Oberfläche des ersten Schafts (260) definiert ist.

13. Verfahren gemäß Anspruch 12, wobei das Anbringen der ersten Dichtung (302) an dem ersten Schaft (260) das Erstrecken des distalen Endabschnitts des ersten Schafts (260) in ein Lumen des Abdichtmechanismus (300), durch die erste Dichtung (302) und in einen Hohlraum (360) des Abdichtmechanismus (300), der durch Wände eines Gehäuses des Abdichtmechanismus (300) zwischen der ersten Dichtung (302) und der zweiten Dichtung (304) definiert ist, umfasst; und/oder
wobei das Anbringen der zweiten Dichtung (304) an dem zweiten Schaft (280) das Erstrecken des distalen Endabschnitts des zweiten Schafts (280) durch und distal zu dem distalen Ende des ersten Schafts (260) und durch die zweite Dichtung (304) umfasst; und/oder
wobei das Anbringen der ersten Dichtung (302) und das Anbringen der zweiten Dichtung (304) das Festziehen der ersten Dichtung (302) um den ersten Schaft (260) und das Festziehen der zweiten Dichtung (304) um den zweiten Schaft (280) derart umfasst, dass das distale Ende des ersten Schafts (260) verschlossen wird.

14. Verfahren gemäß einem der Ansprüche 12 oder 13, wobei das Fließen von Fluid durch den Katheter derart, dass das Fluid nur aus dem zweiten Lumen, das durch den zweiten Schaft (280) definiert ist, ausströmt und daran gehindert wird, aus dem ersten Lumen auszuströmen, das Spülen von Fluid durch den Katheter unter Verwendung von positivem Druck, der auf den Katheter angewendet wird, umfasst.

15. Verfahren gemäß einem der Ansprüche 12 oder 13, wobei das Fließen von Fluid durch den Katheter derart, dass das Fluid nur aus dem zweiten Lumen, das durch den zweiten Schaft (280) definiert ist, ausströmt und daran gehindert wird, aus dem ersten Lumen auszuströmen, das Absaugen von Fluid durch den Katheter unter Verwendung von negativem Druck, der durch ein Aspirationswerkzeug auf ein distales Ende des zweiten Schafts (280) angewendet wird, umfasst.

## Revendications

1. Ensemble, comprenant :
un cathéter comprenant :
une première tige (260) ; et
une seconde tige (280) s'étendant à travers la première tige (260), une première lumière étant définie entre une surface interne de la première tige (260) et une surface externe de la seconde tige (280) ; et
un mécanisme d'étanchéité (300) comprenant :
un premier joint d'étanchéité (302) disposé autour d'une partie extrémité distale de la première tige (260) ;
un second joint d'étanchéité (304) disposé autour d'une partie de la seconde tige (280) qui s'étend de manière distale par rapport à la première tige (260) ; et
une cavité (360) disposée à l'intérieur d'un boîtier du mécanisme d'étanchéité (300) entre le premier joint d'étanchéité (302) et le second joint d'étanchéité (304), une extrémité distale de la première tige (260) étant disposée à l'intérieur de la cavité (360) et la cavité (360) étant fermée hermétiquement de manière fluidique par le premier joint d'étanchéité (302) et le second joint d'étanchéité (304) de telle sorte que le fluide provenant de la première lumière ne peut pas sortir de la cavité (360).

2. Ensemble selon la revendication 1, une extrémité distale de la première lumière étant occluse par la cavité (360), la seconde tige (280) présentant une seconde lumière et une extrémité distale de la seconde lumière étant ouverte et s'étendant de manière distale par rapport au second joint d'étanchéité (304).

3. Ensemble selon soit la revendication 1 soit la revendication 2, le mécanisme d'étanchéité (300) comprenant une marche (358) à l'intérieur de la cavité (360) qui diminue un diamètre de la cavité (360) à partir d'un premier diamètre (326) plus grand adjacent au premier joint d'étanchéité (302) à un second diamètre (354) plus petit adjacent au second joint d'étanchéité (304) et l'extrémité distale de la première tige (260) étant disposée contre la marche (358) ;
de préférence, le boîtier comprenant un premier boîtier d'étanchéité (306) contenant le premier joint d'étanchéité (302) en son sein et un second boîtier d'étanchéité (308) contenant le second joint d'étanchéité (304) en son sein et la marche (358) étant formée sur une surface interne du second boîtier d'étanchéité (308).

4. Ensemble selon l'une quelconque des revendications 1 à 3, le premier joint d'étanchéité (302) étant disposé dans un premier boîtier d'étanchéité (306) du mécanisme d'étanchéité (300) et le second joint d'étanchéité (304) étant disposé dans un second boîtier d'étanchéité (308) du mécanisme d'étanchéité (300), les premier et second boîtiers d'étanchéité (308) étant accouplés l'un à l'autre et la cavité (360) étant définie par une surface interne du premier boîtier d'étanchéité (306) et une surface interne du second boîtier d'étanchéité (308).

5. Ensemble selon la revendication 4, le mécanisme d'étanchéité (300) comprenant en outre un premier élément fileté (336) qui s'interface avec des filetages sur la surface interne du premier boîtier d'étanchéité (306) et qui est conçu pour tourner par rapport au premier boîtier d'étanchéité (306) et serrer le premier joint d'étanchéité (302) autour de la première tige (260) ;
de préférence le mécanisme d'étanchéité (300) comprenant un premier bouton rotatif (340) accouplé au premier élément fileté (336) et conçu pour faire tourner le premier élément fileté (336) de telle sorte qu'il se déplace de manière distale contre le premier joint d'étanchéité (302) et serre le premier joint d'étanchéité (302) autour de la première tige (260).

6. Ensemble selon la revendication 5, le mécanisme d'étanchéité (300) comprenant en outre un second élément fileté (374) qui s'interface avec des filetages sur la surface interne du second boîtier d'étanchéité (308) et qui est conçu pour tourner par rapport au second boîtier d'étanchéité (308) et serrer le second joint d'étanchéité (304) autour de la seconde tige (280) ;
de préférence le mécanisme d'étanchéité (300) comprenant un second bouton rotatif (378) accouplé au second élément fileté (374) et conçu pour faire tourner le second élément fileté (374) de telle sorte qu'il se déplace de manière proximale contre le second joint d'étanchéité (304) et serre le second joint d'étanchéité (304) autour de la seconde tige (280).

7. Ensemble selon la revendication 6, le premier élément fileté (336) présentant une lumière de plus grand diamètre que le second élément fileté (374).

8. Ensemble selon l'une quelconque des revendications 1 ou 2, le premier joint d'étanchéité (302) et le second joint d'étanchéité (304) étant disposés dans le boîtier et la cavité (360) étant définie par une surface interne du boîtier.

9. Ensemble selon la revendication 8, le mécanisme d'étanchéité (300) comprenant en outre un élément fileté (512) qui s'interface avec des filetages sur la surface interne du boîtier au niveau d'une extrémité du boîtier qui est adjacente au premier joint d'étanchéité (302) et l'élément fileté (512) étant conçu pour tourner par rapport au boîtier et serrer le premier joint d'étanchéité (302) autour de la première tige (260) ;
de préférence :
le mécanisme d'étanchéité (300) comprenant un bouton rotatif (510) disposé au niveau d'une extrémité de l'élément fileté (512) et le bouton rotatif (510) étant conçu pour faire tourner l'élément fileté (512) de telle sorte qu'il se déplace de manière distale contre le premier joint d'étanchéité (302) et serre le premier joint d'étanchéité (302) autour de la première tige (260) ; et/ou
le premier joint d'étanchéité (302) étant une garniture compressible (502) et le second joint d'étanchéité (304) étant un joint torique (504).

10. Ensemble selon la revendication 8, le premier joint d'étanchéité (302) étant un joint torique (504) et le second joint d'étanchéité (304) étant un joint torique (504).

11. Ensemble selon l'une quelconque des revendications 1 à 10, le cathéter étant un appareil de pose (200) pour un dispositif d'ancrage (232) et la seconde tige (280) étant conçue pour contenir le dispositif d'ancrage (232) dans une configuration de pose à l'intérieur de la partie extrémité distale de la seconde tige (280).

12. Procédé de rinçage d'un cathéter comprenant :
la fixation d'un premier joint d'étanchéité (302) d'un mécanisme d'étanchéité (300) à une partie extrémité distale d'une première tige (260) d'un cathéter ;
la fixation d'un second joint d'étanchéité (304) d'un mécanisme d'étanchéité (300) à une partie extrémité distale d'une seconde tige (280) du cathéter qui s'étend à travers la première tige (260), la partie extrémité distale de la seconde tige (280) s'étendant de manière distale par rapport à une extrémité distale de la première tige (260) ; et l'écoulement de fluide à travers le cathéter de telle sorte que le fluide s'écoule hors d'une seconde lumière définie par la seconde tige (280) uniquement et est empêché de s'écouler hors d'une première lumière définie entre une surface externe de la seconde tige (280) et une surface interne de la première tige (260).

13. Procédé selon la revendication 12, la fixation du premier joint d'étanchéité (302) à la première tige (260) comportant l'extension de la partie extrémité distale de la première tige (260) dans une lumière du mécanisme d'étanchéité (300), à travers le premier joint d'étanchéité (302) et dans une cavité (360) du mécanisme d'étanchéité (300) qui est définie par des parois d'un boîtier du mécanisme d'étanchéité (300), entre le premier joint d'étanchéité (302) et le second joint d'étanchéité (304) ; et/ou
la fixation du second joint d'étanchéité (304) à la seconde tige (280) comportant l'extension de la partie extrémité distale de la seconde tige (280) à travers l'extrémité distale de la première tige (260) et de manière distale par rapport à celle-ci et à travers le second joint d'étanchéité (304) ; et/ou
la fixation du premier joint d'étanchéité (302) et la fixation du second joint d'étanchéité (304) comportant le serrage du premier joint d'étanchéité (302) autour de la première tige (260) et le serrage du second joint d'étanchéité (304) autour de la seconde tige (280) de telle sorte que l'extrémité distale de la première tige (260) est occluse.

14. Procédé selon l'une quelconque des revendications 12 ou 13, l'écoulement de fluide à travers le cathéter de telle sorte que le fluide s'écoule hors de la seconde lumière définie par la seconde tige (280) uniquement et est empêché de s'écouler hors de la première lumière comportant le rinçage de fluide à travers le cathéter à l'aide d'une pression positive appliquée au cathéter.

15. Procédé selon l'une quelconque des revendications 12 ou 13, l'écoulement de fluide à travers le cathéter de telle sorte que le fluide s'écoule hors de la seconde lumière définie par la seconde tige (280) uniquement et est empêché de s'écouler hors de la première lumière comportant l'aspiration de fluide à travers le cathéter à l'aide d'une pression négative appliquée à une extrémité distale de la seconde tige (280) par un outil d'aspiration.
